# EUROPEAN PATENT SPECIFICATION

(11) **EP 2 217 730 B1**
(45) Date of publication and mention of the grant of the patent: **20.08.2014**
(21) Application number: 08838509.1
(22) Date of filing: 14.10.2008
(51) Int. Cl.: C12Q 1/68, A61K 45/00, G01N 33/566, C07K 14/72, C12N 15/16

(54) **METHODS FOR ASSAYING MC1R VARIANTS AND MITOCHONDRIAL MARKERS IN SKIN SAMPLES**
VERFAHREN ZUM TESTEN VON MC1R-VARIANTEN UND MITOCHONDRIEN-MARKERN IN HAUTPROBEN
PROCÉDÉS POUR ANALYSER DES VARIANTS DE MC1R ET DES MARQUEURS MITOCHONDRIAUX DANS DES ÉCHANTILLONS DE PEAU

(30) Priority: 11.10.2007 WO PCT/CA2007/001790; 15.10.2007 US 999074 P
(43) Date of publication of application: 18.08.2010
(73) Proprietor: Mitomics Inc., Thunder Bay, ON P7A 7T1 (CA)
(72) Inventor: PARR, Ryan, Thunder Bay,Ontario P7G 1J4 (CA); BIRCH-MACHIN, Mark, Newcastle Upon Tyne NE2 4HH (GB); HARBOTTLE, Andrew, Newcastle Upon Tyne NE2 4HH (GB); THAYER, Robert, Thunder Bay, Ontario P7A 3T6 (CA); CREED, Jennifer, Broomfield,Colorado 80023 (US); MAGGRAH, Andrea, Thunder Bay,Ontario P7G 1E5 (CA); ROBINSON, Kerry, Thunder Bay,Ontario P7A 3S9 (CA); DAKUBO, Gabriel, Thunder Bay,Ontario P7J 1H7 (CA); REGULY, Brian, Vancouver,British Columbia V5T 4S8 (CA); MAKI, Katrina, Porcupine,Ontario P0N 1C0 (CA)
(74) Representative: UEXKÜLL & STOLBERG
(86) International application number: PCT/CA2008/001801
(87) International publication number: WO 2009/046535

(56) References cited:
- WO-A1-2006/111029
- WO-A1-2006/111029
- US-A1- 2003 113 906
- US-A1- 2006 084 077
- US-B2- 7 183 057
- HARBOTTLE A ET AL: "Real-time PCR analysis of a 3895 bp mitochondrial DNA deletion in nonmelanoma skin cancer and its use as a quantitative marker for sunlight exposure in human skin." BRITISH JOURNAL OF CANCER 19 JUN 2006 LNKD- PUBMED:16721366, vol. 94, no. 12, 19 June 2006 (2006-06-19) , pages 1887-1893, XP007915295 ISSN: 0007-0920
- ROUZAUD F ET AL: "MC1R and the response of melanocytes to ultraviolet radiation" MUTATION RESEARCH, ELSEVIER, AMSTERDAM, NL LNKD- DOI:10.1016/J.MRFMMM.2004.09.014, vol. 571, no. 1-2, 1 April 2005 (2005-04-01), pages 133-152, XP004770312 ISSN: 0027-5107
- BASTIAENS M T ET AL: "Melanocortin receptor gene variants determine the risk of nonmelanoma skin cancer independently of fair skin and red hair" AMERICAN JOURNAL OF HUMAN GENETICS, AMERICAN SOCIETY OF HUMAN GENETICS, CHICAGO, IL, US, vol. 68, 16 March 2001 (2001-03-16), pages 884-894, XP002310798 ISSN: 0002-9297
- KENNEDY C ET AL: "Melanocortin 1 receptor (MC1R) gene variants are associated with an increased risk for cutaneous melanoma which is largely independent of skin type and hair color." THE JOURNAL OF INVESTIGATIVE DERMATOLOGY AUG 2001 LNKD- PUBMED:11511307, vol. 117, no. 2, August 2001 (2001-08), pages 294-300, XP007915294 ISSN: 0022-202X
- REES, J. L.: 'The Melanocortin 1 Receptor (MC1R): More Than Just Red Hair.' PIGMENT CELL. RES. vol. 13, June 2000, ISSN 0893-5785 pages 135 - 140
- PRITHIVIRAJSINGH, S. ET AL.: 'Accumulation of the Common Mitochondrial DNA Deletion Induced by Ionizing Radiation.' FEBS. LETT. vol. 571, July 2004, ISSN 0014-5793 pages 227 - 232
- WONG, T. H. ET AL.: 'The Relation Between Melanocortin 1 Receptor (MC1R) Variation and the Generation of Phenotypic Diversity inthe Cutaneous Response to Ultraviolet Radiation.' PEPTIDES vol. 26, October 2005, ISSN 0196-9781 pages 1965 - 1971
- REES, J. L. ET AL.: 'The Genetics of Sun Sensitivity in Humans.' AM. J. HUM. GENET. vol. 75, 2004, ISSN 0002-9297 pages 739 - 751
- ESHAGHIAN, A. ET AL.: 'Mitochondrial DNA Deletions Serve as Biomarkers of Aging in the Skin, but are Typically Absent in Nonmelanoma Skin Cancers.' J. INVEST. DERMATOL. vol. 126, February 2006, ISSN 0022-202X pages 336 - 344
- LANDI, M. T. ET AL.: 'MC1R Germline Variants Confer Risk for BRAF- Mutant Melanoma.' SCIENCE vol. 313, 28 July 2006, ISSN 0036 - 80 pages 521 - 522
- BIRCH-MACHIN, M. A. ET AL.: 'Mitochondrial DNA Deletions in Human Skin Reflect Photo-Rather Than Chronologic Aging.' SOC. INVEST. DERMATOL. vol. 110, 1998, pages 149 - 152, XP003003515
- RAY, A. J. ET AL.: 'The Spectrum of Mitochondrial DNA Deletions is a Ubiquitous Marker of Ultraviolet Radiation Exposure in Human Skin.' SOC. INVEST. DERMATOL. vol. 115, 2000, ISSN 0022-202X pages 674 - 679

### SEQUENCE LISTING

<110> Genesis Genomics Inc.
<120> Methods For Assaying MC1R variants And Mitochondrial Markers In skin Samples
<130> 102222/00047
<140> -
   <141> 2008-10-14
<150> PCT/CA2007/001790
   <151> 2007-10-11
<150> US 60/999,074
   <151> 2007-10-15
<160> 12
<170> PatentIn version 3.5
<210> 1
   <211> 12674
   <212> DNA
   <213> Homo sapiens
<400> 1
   gatcacaggt ctatcaccct attaaccact cacgggagct ctccatgcat ttggtatttt      60
   cgtctggggg gtatgcacgc gatagcattg cgagacgctg gagccggagc accctatgtc     120
   gcagtatctg tctttgattc ctgcctcatc ctattattta tcgcacctac gttcaatatt     180
   acaggcgaac atacttacta aagtgtgtta attaattaat gcttgtagga cataataata     240
   acaattgaat gtctgcacag ccactttcca cacagacatc ataacaaaaa atttccacca     300
   aaccccccct cccccgcttc tggccacagc acttaaacac atctctgcca aaccccaaaa     360
   acaaagaacc ctaacaccag cctaaccaga tttcaaattt tatcttttgg cggtatgcac     420
   ttttaacagt caccccccaa ctaacacatt attttcccct cccactccca tactactaat     480
   ctcatcaata caacccccgc ccatcctacc cagcacacac acaccgctgc taaccccata     540
   ccccgaaaat gttggttata cccttcccgt actaattaat cccctggccc aacccgtcat     600
   ctactctacc atctttgcag gcacactcat cacagcgcta agctcgcact gattttttac     660
   ctgagtaggc ctagaaataa acatgctagc ttttattcca gttctaacca aaaaaataaa     720
   ccctcgttcc acagaagctg ccatcaagta tttcctcacg caagcaaccg catccataat     780
   ccttctaata gctatcctct tcaacaatat actctccgga caatgaacca taaccaatac     840
   taccaatcaa tactcatcat taataatcat aatagctata gcaataaaac taggaatagc     900
   cccctttcac ttctgagtcc cagaggttac ccaaggcacc cctctgacat ccggcctgct     960
   tcttctcaca tgacaaaaac tagcccccat ctcaatcata taccaaatct ctccctcact    1020
   aaacgtaagc cttctcctca ctctctcaat cttatccatc atagcaggca gttgaggtgg    1080
   attaaaccaa acccagctac gcaaaatctt agcatactcc tcaattaccc acataggatg 1140
   aataatagca gttctaccgt acaaccctaa cataaccatt cttaatttaa ctatttatat 1200
   tatcctaact actaccgcat tcctactact caacttaaac tccagcacca cgaccctact 1260
   actatctcgc acctgaaaca agctaacatg actaacaccc ttaattccat ccaccctcct 1320
   ctccctagga ggcctgcccc cgctaaccgg ctttttgccc aaatgggcca ttatcgaaga 1380
   attcacaaaa aacaatagcc tcatcatccc caccatcata gccaccatca ccctccttaa 1440
   cctctacttc tacctacgcc taatctactc cacctcaatc acactactcc ccatatctaa 1500
   caacgtaaaa ataaaatgac agtttgaaca tacaaaaccc accccattcc tccccacact 1560
   catcgccctt accacgctac tcctacctat ctcccctttt atactaataa tcttatagaa 1620
   atttaggtta aatacagacc aagagccttc aaagccctca gtaagttgca atacttaatt 1680
   tctgtaacag ctaaggactg caaaacccca ctctgcatca actgaacgca aatcagccac 1740
   tttaattaag ctaagccctt actagaccaa tgggacttaa acccacaaac acttagttaa 1800
   cagctaagca ccctaatcaa ctggcttcaa tctacttctc ccgccgccgg gaaaaaaggc 1860
   gggagaagcc ccggcaggtt tgaagctgct tcttcgaatt tgcaattcaa tatgaaaatc 1920
   acctcggagc tggtaaaaag aggcctaacc cctgtcttta gatttacagt ccaatgcttc 1980
   actcagccat tttacctcac ccccactgat gttcgccgac cgttgactat tctctacaaa 2040
   ccacaaagac attggaacac tatacctatt attcggcgca tgagctggag tcctaggcac 2100
   agctctaagc ctccttattc gagccgagct gggccagcca ggcaaccttc taggtaacga 2160
   ccacatctac aacgttatcg tcacagccca tgcatttgta ataatcttct tcatagtaat 2220
   acccatcata atcggaggct ttggcaactg actagttccc ctaataatcg gtgcccccga 2280
   tatggcgttt ccccgcataa acaacataag cttctgactc ttacctccct ctctcctact 2340
   cctgctcgca tctgctatag tggaggccgg agcaggaaca ggttgaacag tctaccctcc 2400
   cttagcaggg aactactccc accctggagc ctccgtagac ctaaccatct tctccttaca 2460
   cctagcaggt gtctcctcta tcttaggggc catcaatttc atcacaacaa ttatcaatat 2520
   aaaaccccct gccataaccc aataccaaac gcccctcttc gtctgatccg tcctaatcac 2580
   agcagtccta cttctcctat ctctcccagt cctagctgct ggcatcacta tactactaac 2640
   agaccgcaac ctcaacacca ccttcttcga ccccgccgga ggaggagacc ccattctata 2700
   ccaacaccta ttctgatttt tcggtcaccc tgaagtttat attcttatcc taccaggctt 2760
   cggaataatc tcccatattg taacttacta ctccggaaaa aaagaaccat ttggatacat 2820
   aggtatggtc tgagctatga tatcaattgg cttcctaggg tttatcgtgt gagcacacca 2880
   tatatttaca gtaggaatag acgtagacac acgagcatat ttcacctccg ctaccataat 2940
   catcgctatc cccaccggcg tcaaagtatt tagctgactc gccacactcc acggaagcaa 3000
   tatgaaatga tctgctgcag tgctctgagc cctaggattc atctttcttt tcaccgtagg 3060
   tggcctgact ggcattgtat tagcaaactc atcactagac atcgtactac acgacacgta 3120
   ctacgttgta gcccacttcc actatgtcct atcaatagga gctgtatttg ccatcatagg 3180
   aggcttcatt cactgatttc ccctattctc aggctacacc ctagaccaaa cctacgccaa 3240
   aatccatttc actatcatat tcatcggcgt aaatctaact ttcttcccac aacactttct 3300
   cggcctatcc ggaatgcccc gacgttactc ggactacccc gatgcataca ccacatgaaa 3360
   catcctatca tctgtaggct cattcatttc tctaacagca gtaatattaa taattttcat 3420
   gatttgagaa gccttcgctt cgaagcgaaa agtcctaata gtagaagaac cctccataaa 3480
   cctggagtga ctatatggat gccccccacc ctaccacaca ttcgaagaac ccgtatacat 3540
   aaaatctaga caaaaaagga aggaatcgaa ccccccaaag ctggtttcaa gccaacccca 3600
   tggcctccat gactttttca aaaaggtatt agaaaaacca tttcataact ttgtcaaagt 3660
   taaattatag gctaaatcct atatatctta atggcacatg cagcgcaagt aggtctacaa 3720
   gacgctactt cccctatcat agaagagctt atcacctttc atgatcacgc cctcataatc 3780
   attttcctta tctgcttcct agtcctgtat gcccttttcc taacactcac aacaaaacta 3840
   actaatacta acatctcaga cgctcaggaa atagaaaccg tctgaactat cctgcccgcc 3900
   atcatcctag tcctcatcgc cctcccatcc ctacgcatcc tttacataac agacgaggtc 3960
   aacgatccct cccttaccat caaatcaatt ggccaccaat ggtactgaac ctacgagtac 4020
   accgactacg gcggactaat cttcaactcc tacatacttc ccccattatt cctagaacca 4080
   ggcgacctgc gactccttga cgttgacaat cgagtagtac tcccgattga agcccccatt 4140
   cgtataataa ttacatcaca agacgtcttg cactcatgag ctgtccccac attaggctta 4200
   aaaacagatg caattcccgg acgtctaaac caaaccactt tcaccgctac acgaccgggg 4260
   gtatactacg gtcaatgctc tgaaatctgt ggagcaaacc acagtttcat gcccatcgtc 4320
   ctagaattaa ttcccctaaa aatctttgaa atagggcccg tatttaccct atagcacccc 4380
   ctctaccccc tctagagccc actgtaaagc taacttagca ttaacctttt aagttaaaga 4440
   ttaagagaac caacacctct ttacagtgaa atgccccaac taaatactac cgtatggccc 4500
   accataatta cccccatact ccttacacta ttcctcatca cccaactaaa aatattaaac 4560
   acaaactacc acctacctcc ctcaccaaag cccataaaaa taaaaaatta taacaaaccc 4620
   tgagaaccaa aatgaacgaa aatctgttcg cttcattcat tgcccccaca atcctaggcc 4680
   tacccgccgc agtactgatc attctatttc cccctctatt gatccccacc tccaaatatc 4740
   tcatcaacaa ccgactaatc accacccaac aatgactaat caaactaacc tcaaaacaaa 4800
   tgataaccat acacaacact aaaggacgaa cctgatctct tatactagta tccttaatca 4860
   tttttattgc cacaactaac ctcctcggac tcctgcctca ctcatttaca ccaaccaccc 4920
   aactatctat aaacctagcc atggccatcc ccttatgagc gggcacagtg attataggct 4980
   ttcgctctaa gattaaaaat gccctagccc acttcttacc acaaggcaca cctacacccc 5040
   ttatccccat actagttatt atcgaaacca tcagcctact cattcaacca atagccctgg 5100
   ccgtacgcct aaccgctaac attactgcag gccacctact catgcaccta attggaagcg 5160
   ccaccctagc aatatcaacc attaaccttc cctctacact tatcatcttc acaattctaa 5220
   ttctactgac tatcctagaa atcgctgtcg ccttaatcca agcctacgtt ttcacacttc 5280
   tagtaagcct ctacctgcac gacaacacat aatgacccac caatcacatg cctatcatat 5340
   agtaaaaccc agcccatgac ccctaacagg ggccctctca gccctcctaa tgacctccgg 5400
   cctagccatg tgatttcact tccactccat aacgctcctc atactaggcc tactaaccaa 5460
   cacactaacc atataccaat gatggcgcga tgtaacacga gaaagcacat accaaggcca 5520
   ccacacacca cctgtccaaa aaggccttcg atacgggata atcctattta ttacctcaga 5580
   agtttttttc ttcgcaggat ttttctgagc cttttaccac tccagcctag cccctacccc 5640
   ccaattagga gggcactggc ccccaacagg catcaccccg ctaaatcccc tagaagtccc 5700
   actcctaaac acatccgtat tactcgcatc aggagtatca atcacctgag ctcaccatag 5760
   tctaatagaa aacaaccgaa accaaataat tcaagcactg cttattacaa ttttactggg 5820
   tctctatttt accctcctac aagcctcaga gtacttcgag tctcccttca ccatttccga 5880
   cggcatctac ggctcaacat tttttgtagc cacaggcttc cacggacttc acgtcattat 5940
   tggctcaact ttcctcacta tctgcttcat ccgccaacta atatttcact ttacatccaa 6000
   acatcacttt ggcttcgaag ccgccgcctg atactggcat tttgtagatg tggtttgact 6060
   atttctgtat gtctccatct attgatgagg gtcttactct tttagtataa atagtaccgt 6120
   taacttccaa ttaactagtt ttgacaacat tcaaaaaaga gtaataaact tcgccttaat 6180
   tttaataatc aacaccctcc tagccttact actaataatt attacatttt gactaccaca 6240
   actcaacggc tacatagaaa aatccacccc ttacgagtgc ggcttcgacc ctatatcccc 6300
   cgcccgcgtc cctttctcca taaaattctt cttagtagct attaccttct tattatttga     6360
   tctagaaatt gccctccttt tacccctacc atgagcccta caaacaacta acctgccact     6420
   aatagttatg tcatccctct tattaatcat catcctagcc ctaagtctgg cctatgagtg 6480
   actacaaaaa ggattagact gaaccgaatt ggtatatagt ttaaacaaaa cgaatgattt 6540
   cgactcatta aattatgata atcatattta ccaaatgccc ctcatttaca taaatattat 6600
   actagcattt accatctcac ttctaggaat actagtatat cgctcacacc tcatatcctc 6660
   cctactatgc ctagaaggaa taatactatc gctgttcatt atagctactc tcataaccct 6720
   caacacccac tccctcttag ccaatattgt gcctattgcc atactagtct ttgccgcctg 6780
   cgaagcagcg gtgggcctag ccctactagt ctcaatctcc aacacatatg gcctagacta 6840
   cgtacataac ctaaacctac tccaatgcta aaactaatcg tcccaacaat tatattacta 6900
   ccactgacat gactttccaa aaaacacata atttgaatca acacaaccac ccacagccta 6960
   attattagca tcatccctct actatttttt aaccaaatca acaacaacct atttagctgt 7020
   tccccaacct tttcctccga ccccctaaca acccccctcc taatactaac tacctgactc 7080
   ctacccctca caatcatggc aagccaacgc cacttatcca gtgaaccact atcacgaaaa 7140
   aaactctacc tctctatact aatctcccta caaatctcct taattataac attcacagcc 7200
   acagaactaa tcatatttta tatcttcttc gaaaccacac ttatccccac cttggctatc 7260
   atcacccgat gaggcaacca gccagaacgc ctgaacgcag gcacatactt cctattctac 7320
   accctagtag gctcccttcc cctactcatc gcactaattt acactcacaa caccctaggc 7380
   tcactaaaca ttctactact cactctcact gcccaagaac tatcaaactc ctgagccaac 7440
   aacttaatat gactagctta cacaatagct tttatagtaa agatacctct ttacggactc 7500
   cacttatgac tccctaaagc ccatgtcgaa gcccccatcg ctgggtcaat agtacttgcc 7560
   gcagtactct taaaactagg cggctatggt ataatacgcc tcacactcat tctcaacccc 7620
   ctgacaaaac acatagccta ccccttcctt gtactatccc tatgaggcat aattataaca 7680
   agctccatct gcctacgaca aacagaccta aaatcgctca ttgcatactc ttcaatcagc 7740
   cacatagccc tcgtagtaac agccattctc atccaaaccc cctgaagctt caccggcgca 7800
   gtcattctca taatcgccca cgggcttaca tcctcattac tattctgcct agcaaactca 7860
   aactacgaac gcactcacag tcgcatcata atcctctctc aaggacttca aactctactc 7920
   ccactaatag ctttttgatg acttctagca agcctcgcta acctcgcctt accccccact 7980
   attaacctac tgggagaact ctctgtgcta gtaaccacgt tctcctgatc aaatatcact 8040
   ctcctactta caggactcaa catactagtc acagccctat actccctcta catatttacc 8100
   acaacacaat ggggctcact cacccaccac attaacaaca taaaaccctc attcacacga 8160
   gaaaacaccc tcatgttcat acacctatcc cccattctcc tcctatccct caaccccgac 8220
   atcattaccg ggttttcctc ttgtaaatat agtttaacca aaacatcaga ttgtgaatct 8280
   gacaacagag gcttacgacc ccttatttac cgagaaagct cacaagaact gctaactcat 8340
   gcccccatgt ctaacaacat ggctttctca acttttaaag gataacagct atccattggt 8400
   cttaggcccc aaaaattttg gtgcaactcc aaataaaagt aataaccatg cacactacta 8460
   taaccaccct aaccctgact tccctaattc cccccatcct taccaccctc gttaacccta 8520
   acaaaaaaaa ctcatacccc cattatgtaa aatccattgt cgcatccacc tttattatca 8580
   gtctcttccc cacaacaata ttcatgtgcc tagaccaaga agttattatc tcgaactgac 8640
   actgagccac aacccaaaca acccagctct ccctaagctt caaactagac tacttctcca 8700
   taatattcat ccctgtagca ttgttcgtta catggtccat catagaattc tcactgtgat 8760
   atataaactc agacccaaac attaatcagt tcttcaaata tctactcatc ttcctaatta 8820
   ccatactaat cttagttacc gctaacaacc tattccaact gttcatcggc tgagagggcg 8880
   taggaattat atccttcttg ctcatcagtt gatgatacgc ccgagcagat gccaacacag 8940
   cagccattca agcaatccta tacaaccgta tcggcgatat cggtttcatc ctcgccttag 9000
   catgatttat cctacactcc aactcatgag acccacaaca aatagccctt ctaaacgcta 9060
   atccaagcct caccccacta ctaggcctcc tcctagcagc agcaggcaaa tcagcccaat 9120
   taggtctcca cccctgactc ccctcagcca tagaaggccc caccccagtc tcagccctac 9180
   tccactcaag cactatagtt gtagcaggaa tcttcttact catccgcttc caccccctag 9240
   cagaaaatag cccactaatc caaactctaa cactatgctt aggcgctatc accactctgt 9300
   tcgcagcagt ctgcgccctt acacaaaatg acatcaaaaa aatcgtagcc ttctccactt 9360
   caagtcaact aggactcata atagttacaa tcggcatcaa ccaaccacac ctagcattcc 9420
   tgcacatctg tacccacgcc ttcttcaaag ccatactatt tatgtgctcc gggtccatca 9480
   tccacaacct taacaatgaa caagatattc gaaaaatagg aggactactc aaaaccatac 9540
   ctctcacttc aacctccctc accattggca gcctagcatt agcaggaata cctttcctca 9600
   caggtttcta ctccaaagac cacatcatcg aaaccgcaaa catatcatac acaaacgcct 9660
   gagccctatc tattactctc atcgctacct ccctgacaag cgcctatagc actcgaataa 9720
   ttcttctcac cctaacaggt caacctcgct tccccaccct tactaacatt aacgaaaata 9780
   accccaccct actaaacccc attaaacgcc tggcagccgg aagcctattc gcaggatttc 9840
   tcattactaa caacatttcc cccgcatccc ccttccaaac aacaatcccc ctctacctaa 9900
   aactcacagc cctcgctgtc actttcctag gacttctaac agccctagac ctcaactacc 9960
   taaccaacaa acttaaaata aaatccccac tatgcacatt ttatttctcc aacatactcg 10020
   gattctaccc tagcatcaca caccgcacaa tcccctatct aggccttctt acgagccaaa 10080
   acctgcccct actcctccta gacctaacct gactagaaaa gctattacct aaaacaattt 10140
   cacagcacca aatctccacc tccatcatca cctcaaccca aaaaggcata attaaacttt 10200
   acttcctctc tttcttcttc ccactcatcc taaccctact cctaatcaca taacctattc 10260
   ccccgagcaa tctcaattac aatatataca ccaacaaaca atgttcaacc agtaactact 10320
   actaatcaac gcccataatc atacaaagcc cccgcaccaa taggatcctc ccgaatcaac 10380
   cctgacccct ctccttcata aattattcag cttcctacac tattaaagtt taccacaacc 10440
   accaccccat catactcttt cacccacagc accaatccta cctccatcgc taaccccact 10500
   aaaacactca ccaagacctc aacccctgac ccccatgcct caggatactc ctcaatagcc 10560
   atcgctgtag tatatccaaa gacaaccatc attcccccta aataaattaa aaaaactatt 10620
   aaacccatat aacctccccc aaaattcaga ataataacac acccgaccac accgctaaca 10680
   atcaatacta aacccccata aataggagaa ggcttagaag aaaaccccac aaaccccatt 10740
   actaaaccca cactcaacag aaacaaagca tacatcatta ttctcgcacg gactacaacc 10800
   acgaccaatg atatgaaaaa ccatcgttgt atttcaacta caagaacacc aatgacccca 10860
   atacgcaaaa ctaaccccct aataaaatta attaaccact cattcatcga cctccccacc 10920
   ccatccaaca tctccgcatg atgaaacttc ggctcactcc ttggcgcctg cctgatcctc 10980
   caaatcacca caggactatt cctagccatg cactactcac cagacgcctc aaccgccttt 11040
   tcatcaatcg cccacatcac tcgagacgta aattatggct gaatcatccg ctaccttcac 11100
   gccaatggcg cctcaatatt ctttatctgc ctcttcctac acatcgggcg aggcctatat 11160
   tacggatcat ttctctactc agaaacctga aacatcggca ttatcctcct gcttgcaact 11220
   atagcaacag ccttcatagg ctatgtcctc ccgtgaggcc aaatatcatt ctgaggggcc 11280
   acagtaatta caaacttact atccgccatc ccatacattg ggacagacct agttcaatga 11340
   atctgaggag gctactcagt agacagtccc accctcacac gattctttac ctttcacttc 11400
   atcttgccct tcattattgc agccctagca acactccacc tcctattctt gcacgaaacg 11460
   ggatcaaaca accccctagg aatcacctcc cattccgata aaatcacctt ccacccttac 11520
   tacacaatca aagacgccct cggcttactt ctcttccttc tctccttaat gacattaaca 11580
   ctattctcac cagacctcct aggcgaccca gacaattata ccctagccaa ccccttaaac 11640
   acccctcccc acatcaagcc cgaatgatat ttcctattcg cctacacaat tctccgatcc 11700
   gtccctaaca aactaggagg cgtccttgcc ctattactat ccatcctcat cctagcaata 11760
   atccccatcc tccatatatc caaacaacaa agcataatat ttcgcccact aagccaatca 11820
   ctttattgac tcctagccgc agacctcctc attctaacct gaatcggagg acaaccagta 11880
   agctaccctt ttaccatcat tggacaagta gcatccgtac tatacttcac aacaatccta 11940
   atcctaatac caactatctc cctaattgaa aacaaaatac tcaaatgggc ctgtccttgt 12000
   agtataaact aatacaccag tcttgtaaac cggagatgaa aacctttttc caaggacaaa 12060
   tcagagaaaa agtctttaac tccaccatta gcacccaaag ctaagattct aatttaaact 12120
   attctctgtt ctttcatggg gaagcagatt tgggtaccac ccaagtattg actcacccat 12180
   caacaaccgc tatgtatttc gtacattact gccagccacc atgaatattg tacggtacca 12240
   taaatacttg accacctgta gtacataaaa acccaatcca catcaaaacc ccctccccat 12300
   gcttacaagc aagtacagca atcaaccctc aactatcaca catcaactgc aactccaaag 12360
   ccacccctca cccactagga taccaacaaa cctacccacc cttaacagta catagtacat 12420
   aaagccattt accgtacata gcacattaca gtcaaatccc ttctcgtccc catggatgac 12480
   ccccctcaga taggggtccc ttgaccacca tcctccgtga aatcaatatc ccgcacaaga 12540
   gtgctactct cctcgctccg ggcccataac acttgggggt agctaaagtg aactgtatcc 12600
   gacatctggt tcctacttca gggtcataaa gcctaaatag cccacacgtt ccccttaaat 12660
   aagacatcac gatg 12674
<210> 2
   <211> 3895
   <212> DNA
   <213> Homo sapiens
<220>
   <221> misc_feature
   <222> (2559)..(2559)
   <223> n is a, c, g, or t
<400> 2
   ccaaccaaac cccaaagaca ccccccacag tttatgtagc ttacctcctc aaagcaatac 60
   actgaaaatg tttagacggg ctcacatcac cccataaaca aataggtttg gtcctagcct 120
   ttctattagc tcttagtaag attacacatg caagcatccc cgttccagtg agttcaccct 180
   ctaaatcacc acgatcaaaa ggaacaagca tcaagcacgc agcaatgcag ctcaaaacgc 240
   ttagcctagc cacaccccca cgggaaacag cagtgattaa cctttagcaa taaacgaaag 300
   tttaactaag ctatactaac cccagggttg gtcaatttcg tgccagccac cgcggtcaca 360
   cgattaaccc aagtcaatag aagccggcgt aaagagtgtt ttagatcacc ccctccccaa 420
   taaagctaaa actcacctga gttgtaaaaa actccagttg acacaaaata gactacgaaa 480
   gtggctttaa catatctgaa cacacaatag ctaagaccca aactgggatt agatacccca 540
   ctatgcttag ccctaaacct caacagttaa atcaacaaaa ctgctcgcca gaacactacg 600
   agccacagct taaaactcaa aggacctggc ggtgcttcat atccctctag aggagcctgt 660
   tctgtaatcg ataaaccccg atcaacctca ccacctcttg ctcagcctat ataccgccat 720
   cttcagcaaa ccctgatgaa ggctacaaag taagcgcaag tacccacgta aagacgttag 780
   gtcaaggtgt agcccatgag gtggcaagaa atgggctaca ttttctaccc cagaaaacta 840
   cgatagccct tatgaaactt aagggtcgaa ggtggattta gcagtaaact aagagtagag 900
   tgcttagttg aacagggccc tgaagcgcgt acacaccgcc cgtcaccctc ctcaagtata 960
   cttcaaagga catttaacta aaacccctac gcatttatat agaggagaca agtcgtaaca 1020
   tggtaagtgt actggaaagt gcacttggac gaaccagagt gtagcttaac acaaagcacc 1080
   caacttacac ttaggagatt tcaacttaac ttgaccgctc tgagctaaac ctagccccaa 1140
   acccactcca ccttactacc agacaacctt agccaaacca tttacccaaa taaagtatag 1200
   gcgatagaaa ttgaaacctg gcgcaataga tatagtaccg caagggaaag atgaaaaatt 1260
   ataaccaagc ataatatagc aaggactaac ccctatacct tctgcataat gaattaacta 1320
   gaaataactt tgcaaggaga gccaaagcta agacccccga aaccagacga gctacctaag 1380
   aacagctaaa agagcacacc cgtctatgta gcaaaatagt gggaagattt ataggtagag 1440
   gcgacaaacc taccgagcct ggtgatagct ggttgtccaa gatagaatct tagttcaact 1500
   ttaaatttgc ccacagaacc ctctaaatcc ccttgtaaat ttaactgtta gtccaaagag 1560
   gaacagctct ttggacacta ggaaaaaacc ttgtagagag agtaaaaaat ttaacaccca 1620
   tagtaggcct aaaagcagcc accaattaag aaagcgttca agctcaacac ccactaccta 1680
   aaaaatccca aacatataac tgaactcctc acacccaatt ggaccaatct atcaccctat 1740
   agaagaacta atgttagtat aagtaacatg aaaacattct cctccgcata agcctgcgtc 1800
   agattaaaac actgaactga caattaacag cccaatatct acaatcaacc aacaagtcat 1860
   tattaccctc actgtcaacc caacacaggc atgctcataa ggaaaggtta aaaaaagtaa 1920
   aaggaactcg gcaaatctta ccccgcctgt ttaccaaaaa catcacctct agcatcacca 1980
   gtattagagg caccgcctgc ccagtgacac atgtttaacg gccgcggtac cctaaccgtg 2040
   caaaggtagc ataatcactt gttccttaaa tagggacctg tatgaatggc tccacgaggg 2100
   ttcagctgtc tcttactttt aaccagtgaa attgacctgc ccgtgaagag gcgggcataa 2160
   cacagcaaga cgagaagacc ctatggagct ttaatttatt aatgcaaaca gtacctaaca 2220
   aacccacagg tcctaaacta ccaaacctgc attaaaaatt tcggttgggg cgacctcgga 2280
   gcagaaccca acctccgagc agtacatgct aagacttcac cagtcaaagc gaactactat 2340
   actcaattga tccaataact tgaccaacgg aacaagttac cctagggata acagcgcaat 2400
   cctattctag agtccatatc aacaataggg tttacgacct cgatgttgga tcaggacatc 2460
   ccgatggtgc agccgctatt aaaggttcgt ttgttcaacg attaaagtcc tacgtgatct 2520
   gagttcagac cggagtaatc caggtcggtt tctatctanc ttcaaattcc tccctgtacg 2580
   aaaggacaag agaaataagg cctacttcac aaagcgcctt cccccgtaaa tgatatcatc 2640
   tcaacttagt attataccca cacccaccca agaacagggt ttgttaagat ggcagagccc 2700
   ggtaatcgca taaaacttaa aactttacag tcagaggttc aattcctctt cttaacaaca 2760
   tacccatggc caacctccta ctcctcattg tacccattct aatcgcaatg gcattcctaa 2820
   tgcttaccga acgaaaaatt ctaggctata tacaactacg caaaggcccc aacgttgtag 2880
   gcccctacgg gctactacaa cccttcgctg acgccataaa actcttcacc aaagagcccc 2940
   taaaacccgc cacatctacc atcaccctct acatcaccgc cccgacctta gctctcacca 3000
   tcgctcttct actatgaacc cccctcccca tacccaaccc cctggtcaac ctcaacctag 3060
   gcctcctatt tattctagcc acctctagcc tagccgttta ctcaatcctc tgatcagggt 3120
   gagcatcaaa ctcaaactac gccctgatcg gcgcactgcg agcagtagcc caaacaatct 3180
   catatgaagt caccctagcc atcattctac tatcaacatt actaataagt ggctccttta 3240
   acctctccac ccttatcaca acacaagaac acctctgatt actcctgcca tcatgaccct 3300
   tggccataat atgatttatc tccacactag cagagaccaa ccgaaccccc ttcgaccttg 3360
   ccgaagggga gtccgaacta gtctcaggct tcaacatcga atacgccgca ggccccttcg 3420
   ccctattctt catagccgaa tacacaaaca ttattataat aaacaccctc accactacaa 3480
   tcttcctagg aacaacatat gacgcactct cccctgaact ctacacaaca tattttgtca 3540
   ccaagaccct acttctaacc tccctgttct tatgaattcg aacagcatac ccccgattcc 3600
   gctacgacca actcatacac ctcctatgaa aaaacttcct accactcacc ctagcattac 3660
   ttatatgata tgtctccata cccattacaa tctccagcat tccccctcaa acctaagaaa 3720
   tatgtctgat aaaagagtta ctttgataga gtaaataata ggagcttaaa cccccttatt 3780
   tctaggacta tgagaatcga acccatccct gagaatccaa aattctccgt gccacctatc 3840
   acaccccatc ctaaagtaag gtcagctaaa taagctatcg ggcccatacc ccgaa 3895
<210> 3
   <211> 2360
   <212> DNA
   <213> Homo sapiens
<400> 3
   gagagggcag gtcccgggga agctccggac tcctagaggg gcggccaggt gggggccctg 60
   gtgaccagga cagactgtgg tgttttttaa cgtaaaggag atccgcggtg tgagggaccc 120
   cctgggtcct gcacgccgcc tggtggcagg ccgggccatg gtgggtgctc acgcccccgg 180
   catgtggccg ccctcagtgg gaggggctct gagaacgact ttttaaaacg cagagaaaag 240
   ctccattctt cccaggacct cagcgcagcc ctggcccagg aaggcaggag acagaggcca 300
   ggacggtcca gaggtgtcga aatgtcctgg ggacctgagc agcagccacc agggaagagg 360
   cagggaggga gctgaggacc aggcttggtt gtgagaatcc ctgagcccag gcggtagatg 420
   ccaggaggtg tctggactgg ctgggccatg cctgggctga cctgtccagc cagggagagg 480
   gtgtgagggc agatctgggg gtgcccagat ggaaggaggc aggcatgggg gacacccaag 540
   gccccctggc agcaccatga actaagcagg acacctggag gggaagaact gtggggacct 600
   ggaggcctcc aacgactcct tcctgcttcc tggacaggac tatggctgtg cagggatccc 660
   agagaagact tctgggctcc ctcaactcca cccccacagc catcccccag ctggggctgg 720
   ctgccaacca gacaggagcc cggtgcctgg aggtgtccat ctctgacggg ctcttcctca 780
   gcctggggct ggtgagcttg gtggagaacg cgctggtggt ggccaccatc gccaagaacc 840
   ggaacctgca ctcacccatg tactgcttca tctgctgcct ggccttgtcg gacctgctgg 900
   tgagcgggag caacgtgctg gagacggccg tcatcctcct gctggaggcc ggtgcactgg 960
   tggcccgggc tgcggtgctg cagcagctgg acaatgtcat tgacgtgatc acctgcagct 1020
   ccatgctgtc cagcctctgc ttcctgggcg ccatcgccgt ggaccgctac atctccatct 1080
   tctacgcact gcgctaccac agcatcgtga ccctgccgcg ggcgcggcga gccgttgcgg 1140
   ccatctgggt ggccagtgtc gtcttcagca cgctcttcat cgcctactac gaccacgtgg 1200
   ccgtcctgct gtgcctcgtg gtcttcttcc tggctatgct ggtgctcatg gccgtgctgt 1260
   acgtccacat gctggcccgg gcctgccagc acgcccaggg catcgcccgg ctccacaaga 1320
   ggcagcgccc ggtccaccag ggctttggcc ttaaaggcgc tgtcaccctc accatcctgc 1380
   tgggcatttt cttcctctgc tggggcccct tcttcctgca tctcacactc atcgtcctct 1440
   gccccgagca ccccacgtgc ggctgcatct tcaagaactt caacctcttt ctcgccctca 1500
   tcatctgcaa tgccatcatc gaccccctca tctacgcctt ccacagccag gagctccgca 1560
   ggacgctcaa ggaggtgctg acatgctcct ggtgagcgcg gtgcacgcgg ctttaagtgt 1620
   gctgggcaga gggaggtggt gatattgtgt ggtctggttc ctgtgtgacc ctgggcagtt 1680
   ccttacctcc ctggtccccg tttgtcaaag aggatggact aaatgatctc tgaaagtgtt 1740
   gaagcgcgga cccttctggg tccagggagg ggtccctgca aaactccagg caggacttct 1800
   caccagcagt cgtggggaac ggaggaggac atggggaggt tgtggggcct caggctccgg 1860
   gcaccagggg ccaacctcag gctcctaaag agacattttc cgcccactcc tgggacactc 1920
   cgtctgctcc aatgactgag cagcatccac cccaccccat ctttgctgcc agctctcagg 1980
   accgtgccct cgtcagctgg gatgtgaagt ctctgggtgg aagtgtgtgc caagagctac 2040
   tcccacagca gccccaggag aaggggcttt gtgaccagaa agcttcatcc acagccttgc 2100
   agcggctcct gcaaaaggag gtgaaatccc tgcctcaggc caagggacca ggtttgcagg 2160
   agccccccta gtggtatggg gctgagccct cctgagggcc ggttctaagg ctcagactgg 2220
   gcactggggc ctcagcctgc tttcctgcag cagtcgccca agcagacagc cctggcaaat 2280
   gcctgactca gtgaccagtg cctgtgagca tggggccagg aaagtctggt aataaatgtg 2340
   actcagcatc acccacctta 2360
<210> 4
   <211> 3115
   <212> RNA
   <213> Homo sapiens
<400> 4
   agacgcaguc uucagcaagg aagugcuggg aacgcccugg agugaaccca ggaagaugcc 60
   ugcagugggu gccagggccc cucuccaccg ucccugcugg gcuucggggc cacgcccgac 120
   ugcugugaac ggccugcgga gcaccacgug cgacggcugg aggcgagagg ucugccuuug      180
   auguggcugu uggugcaggg ccuguggugc cuuccgcagc ggaaauggcg cgccgcccgg     240
   ggagggcggg agcagcgucc cgggugcccc ugugaggaug agcgacgaga ugacuggagg 300
   gucccugaag accucacuag ggugccccca gccgguccgc ucccaggaag cgacaccccc 360
   acagccccag ggcugcagcu gagggggucg ccacucuggc ugggcgaggc ugggcccuug 420
   ggggcaggcg ccagaguggc cucaggcucu acaagaugcc ugaaaacacc aaccucucca 480
   gggcucacua gcauuggacg cuuucacgcu cugcccuggc cggaagcccc cucaccccgc 540
   gcgaugugca aacuccugca gggcucacuc aguuuccaga acuuuaauua uuggaaaguu 600
   cucccugguc cagcccccaa aucugccgug aacguugaca gcugaguugc ugcuccaugc 660
   gugcuuuggc ugagagcaga ggggaccccu guccucccug agcugcugac gaggggaggg 720
   gugaagggug gggccucugg agagggcagg ucccggggaa gcuccggacu ccuagagggg 780
   cggccaggug ggggcccugg ugaccaggac agacuguggu guuuuuuaac guaaaggaga 840
   uccgcggugu gagggacccc cuggguccug cacgccgccu gguggcaggc cgggccaugg 900
   ugggugcuca cgcccccggc auguggccgc ccucaguggg aggggcucug agaacgacuu 960
   uuuaaaacgc agagaaaagc uccauucuuc ccaggaccuc agcgcagccc uggcccagga 1020
   aggcaggaga cagaggccag gacgguccag aggugucgaa auguccuggg gaccugagca 1080
   gcagccacca gggaagaggc agggagggag cugaggacca ggcuugguug ugagaauccc 1140
   ugagcccagg cgguagaugc caggaggugu cuggacuggc ugggccaugc cugggcugac 1200
   cuguccagcc agggagaggg ugugagggca gaucuggggg ugcccagaug gaaggaggca 1260
   ggcauggggg acacccaagg cccccuggca gcaccaugaa cuaagcagga caccuggagg 1320
   ggaagaacug uggggaccug gaggccucca acgacuccuu ccugcuuccu ggacaggacu 1380
   auggcugugc agggauccca gagaagacuu cugggcuccc ucaacuccac ccccacagcc 1440
   aucccccagc uggggcuggc ugccaaccag acaggagccc ggugccugga gguguccauc   1500
   ucugacgggc ucuuccucag ccuggggcug gugagcuugg uggagaacgc gcugguggug 1560
   gccaccaucg ccaagaaccg gaaccugcac ucacccaugu acugcuucau cugcugccug 1620
   gccuugucgg accugcuggu gagcgggagc aacgugcugg agacggccgu cauccuccug 1680
   cuggaggccg gugcacuggu ggcccgggcu gcggugcugc agcagcugga caaugucauu 1740
   gacgugauca ccugcagcuc caugcugucc agccucugcu uccugggcgc caucgccgug 1800
   gaccgcuaca ucuccaucuu cuacgcacug cgcuaccaca gcaucgugac ccugccgcgg 1860
   gcgcggcgag ccguugcggc caucugggug gccagugucg ucuucagcac gcucuucauc 1920
   gccuacuacg accacguggc cguccugcug ugccucgugg ucuucuuccu ggcuaugcug 1980
   gugcucaugg ccgugcugua cguccacaug cuggcccggg ccugccagca cgcccagggc 2040
   aucgcccggc uccacaagag gcagcgcccg guccaccagg gcuuuggccu uaaaggcgcu 2100
   gucacccuca ccauccugcu gggcauuuuc uuccucugcu ggggccccuu cuuccugcau 2160
   cucacacuca ucguccucug ccccgagcac cccacgugcg gcugcaucuu caagaacuuc 2220
   aaccucuuuc ucgcccucau caucugcaau gccaucaucg acccccucau cuacgccuuc 2280
   cacagccagg agcuccgcag gacgcucaag gaggugcuga caugcuccug gugagcgcgg 2340
   ugcacgcggc uuuaagugug cugggcagag ggagguggug auauugugug gucugguucc   2400
   ugugugaccc ugggcaguuc cuuaccuccc ugguccccgu uugucaaaga ggauggacua 2460
   aaugaucucu gaaaguguug aagcgcggac ccuucugggu ccagggaggg gucccugcaa 2520
   aacuccaggc aggacuucuc accagcaguc guggggaacg gaggaggaca uggggagguu 2580
   guggggccuc aggcuccggg caccaggggc caaccucagg cuccuaaaga gacauuuucc 2640
   gcccacuccu gggacacucc gucugcucca augacugagc agcauccacc ccaccccauc 2700
   uuugcugcca gcucucagga ccgugcccuc gucagcuggg augugaaguc ucugggugga 2760
   agugugugcc aagagcuacu cccacagcag ccccaggaga aggggcuuug ugaccagaaa 2820
   gcuucaucca cagccuugca gcggcuccug caaaaggagg ugaaaucccu gccucaggcc 2880
   aagggaccag guuugcagga gccccccuag ugguaugggg cugagcccuc cugagggccg 2940
   guucuaaggc ucagacuggg cacuggggcc ucagccugcu uuccugcagc agucgcccaa 3000
   gcagacagcc cuggcaaaug ccugacucag ugaccagugc cugugagcau ggggccagga 3060
   aagucuggua auaaauguga cucagcauca cccaccuuaa aaaaaaaaaa aaaaa 3115
<210> 5
   <211> 28
   <212> DNA
   <213> Artificial Sequence
<220>
   <223> 3895 mtDNA deletion forward primer
<400> 5
   ctgctaaccc cataccccga aaatgttg 28
<210> 6
   <211> 31
   <212> DNA
   <213> Artificial Sequence
<220>
   <223> 3895 mtDNA deletion reverse primer
<400> 6
   gaaggattat ggatgcggtt gcttgcgtga g 31
<210> 7
   <211> 21
   <212> DNA
   <213> Artificial Sequence
<220>
   <223> mtDNA genome forward primer
<400> 7
   cgttccagtg agttcaccct c 21
<210> 8
   <211> 23
   <212> DNA
   <213> Artificial Sequence
<220>
   <223> mtDNA genome reverse primer
<400> 8
   cactctttac gccggcttct att 23
<210> 9
   <211> 20
   <212> DNA
   <213> Artificial sequence
<220>
   <223> MC1R-294 forward primer
<400> 9 -
   cgccctcatc atctgcaatg 20
<210> 10
   <211> 19
   <212> DNA
   <213> Artificial Sequence
<220>
   <223> MC1R-294 reverse primer
<400> 10
   ggctgtggaa ggcgtagat 19
<210> 11
   <211> 16
   <212> DNA
   <213> Artificial Sequence
<220>
   <223> MC1R-294V1 VIC probe
<400> 11
   ccatcatcga ccccct 16
<210> 12
   <211> 16
   <212> DNA
   <213> Artificial Sequence
<220>
   <223> MC1R-294M1 FAM probe
<400> 12
   ccatcatcca ccccct 16

## Description

### FIELD OF THE ENVIENTION

The present invention relates to methods for predicting, diagnosing and monitoring skin states and skin diseases. In particular, the present invention pertains to methods coupling non-invasive skin sampling techniques with assays for determining mitochondrial mutations and Melanocortin 1 Receptor (MC1R) variants for use as a comprehensive tool for predicating and monitoring disease, photoageing and ultraviolet radiation (UVR) damage. The methods are also useful for assessing the effectiveness of and response to therapeutic agents, skin care products and treatment regimes.

### BACKGROUND OF THE INVENTION

Skin disease represents a major health care challenge in today's world. With more than one million new cases of skin cancer diagnosed each year in the United States (National Cancer Institute, www.cancer.gov), predicting and diagnosing skin disease are important aspects of its management. Current diagnostic methods rely mainly on visible observations and biopsies. Detection methods that rely on visible observations, however, are not necessarily effective for diagnosing skin states or diseases, and do not detect risk of disease until after clinical manifestation. Furthermore, invasive methods such as biopsies, are not only traumatic for a subject being tested, they also increased the chances of infection. These methods must also be performed by a medical practitioner in order to be safely conducted, and typically do not provide an enriched sample of cells on the surface of skin, which are the cells generally involved in a reaction.

Non-invasive methods of diagnosing and monitoring skin states and diseases, therefore, represent important tools for patient management, and for assessing the efficacy of existing and new therapeutic agents, skin care products and skin care regimes. Furthermore, these methods may provide important information regarding the specific genetic changes underlying a subject's skin state, as well as their genetic predisposition for developing a skin disease. Identifying these genetic changes identifies potential drug targets and preventative measures, and may be critical in determining whether a person will actually respond to a particular therapeutic agent, skin care product or regime. As well, detection and diagnosis methods are important in assessing the safety of such therapies, products and measures.

### Ultraviolet Radiation (UVR) Damage

Unknown or poorly quantifiable factors often contribute to skin damage as a result of ultraviolet radiation (UVR). Known factors include skin colour, frequency and severity of UVR exposure, melanin production, ratios of eumelanin to pheomelanin, and sunscreen use among others. Behavioural factors play a large role in the severity and level of damage to skin from UVR but are extremely difficult to assess clinically as they are dependent upon the accuracy of self-reporting and often a flawed awareness of a patient's sun lifestyle habits. Many individuals who use sunscreen regularly also use it improperly, failing to apply it in sufficient quantity or to reapply at the recommended intervals, creating a false sense of protection that can lead to increased exposure to UVR.

These factors among others necessitate the availability of a tool to closely monitor the success and appropriateness of the preventative measures and therapies being advised to prevent UVR damage to the skin, with consequences in the spectrum from premature photoaging to increasing skin cancer risk. In an effort to fully evaluate an individual's skin state, and as a result their risk of photoaging and skin cancer, it is imperative that health care practitioners are provided with as much insight as possible to understand and communicate their patient's risk factors. It would, therefore, be desirable to have a diagnostic method for assessing an individual's genetic predisposition to the damaging effects of UVR, as well as the identification of risk factors associated with an individual's particular sun lifestyle habits. Such a method may include the collection of a DNA sample from the epidermal or dermal layers of an individual's skin for quantification of mitochondrial biomarkers indicative of UVR damage, as well as Melanocortin 1 Receptor (MC1R) genotyping, to identify variants involved in the control of melanogenesis. The combined use of both tests in tandem provides a comprehensive tool for health care providers to monitor, advise, and treat patients by considering both a patient's genetic predisposition to photoaging or cancer, and the result of the patient's ongoing exposure to ultraviolet radiation.

### Mitochondrial deletions associated with UVR

Human skin tissue is highly complex and comprises numerous cell types. Accordingly, the identification of human skin cell biomarkers is of particular importance. To determine a reliable marker of cumulative UVR exposure in human skin, the inventors and others have examined the novel idea of using mitochondrial DNA (mtDNA), rather than nuclear DNA, as a biomarker of UV-induced DNA damage (Pang et al., 1994; Berneburg et al., 1997; Birch-Machin et al., 1998; Birch-Machin, 2000).

The use of mtDNA damage as a biomarker for cumulative sun-exposure in human skin is a relatively new field of research and previous work has simply compared mtDNA damage to distinguish between sun-protected and sun-exposed skin (Pang et al., 1994; Berneburg et al., 1997; Birch-Machin et al., 1998). This approach is limited because non-melanoma skin cancer (NMSC) is predominantly formed on body sites which are "usually" exposed to the sun when outdoors as opposed to sites that are "occasionally" exposed to the sun (Armstrong, 2004).

In the present Applicant's co-pending PCT application bearing publication no. WO/06/111029, a 3895 bp deletion in human mitochondrial DNA (mtDNA) was identified as a biomarker of UV-induced DNA damage. This deletion was identified in the minor arc spanning nucleotides 547-4443. This deletion had previously been associated with Kearns Sayre Syndrome and Chronic Progressive External Opthalmoplegia (Moraes et al., 1995).

Examples in PCT publication no.WO/06/111029 demonstrate that that the frequency of occurrence of the 3895 bp mtDNA deletion is significantly different between body sites that are "usually" versus "occasionally" exposed to the sun. In addition, the examples demonstrated a link between the etiology of the 3895 bp deletion and the UVR component of sunlight by inducing the 3895 bp deletion *in vitro* with repetitive sub-lethal doses of a UVA+UVB light source.

Importantly, skin samples analysed in WO/06/111029 were not assessed with regard to the individual's genetic predisposition to sun damage and risk for skin cancer. In addition, these samples were obtained by painful methods of skin collection previously known in the art.

### Melanocortin 1 Receptor (MC1R) Genotyping

The melanocortin-1 receptor gene *(MC1R)* encodes a membrane-bound receptor protein that is central to melanin synthesis. The coding region of *MC1R* is highly polymorphic and associations of variants with pigmentation phenotypes and risk for melanoma and non-melanoma skin cancer have been reported (Rees, Pigment Cell Research, Vol. 13(3), 135-140(6), June 2000; Kanetsky et al. Cancer Epidemiology Biomarkers & Prevention Vol. 13, 808-819, May 2004). The incidence rate of melanoma is greatest in fair-skinned, sun sensitive individuals, suggesting that the ability to respond to UV exposure, by increased synthesis of melanin is an important factor in melanoma defense. Family studies have shown that individuals with fair skin and red hair harbor functionally significant changes in both MC1R alleles. The alleles D84E, R142H, R151C, R160H and D294H have high penetrance for red hair and fair skin. Two lower penetrance alleles (V60L, V92M) are also common factors in melanoma risk.

MC 1 R is, therefore, a major determinant of sun sensitivity and a genetic risk factor for skin cancer. Assessing DNA or amino acid sequence of the Melanocortin 1 Receptor (*MCIR*) gene to identify whether certain variants which are associated with increased sun sensitivity, susceptibility to DNA damage and increased skin cancer risk are present provides a valuable tool to identify patients with greater risk who are in need of more aggressive monitoring and treatment measures. Evaluation of phenotypic characteristics associated with sun sensitivity alone would not be able to elucidate this increased risk.

### Skin sampling

Current methods for the collection of skin samples for use in the diagnosis or characterization of diseases, such as skin cancer, include invasive or painful methods that can cause substantial discomfort to the individual being tested. Examples of current methods for the collection of skin samples include punch biopsy, tapelift, and surgical excision. In addition to the discomfort caused by the current methods for skin collection, these methods must also be performed by a medical practitioner in order to be safely conducted.

Such invasive techniques have other disadvantages including risk of infection, inconvenience of sample collection, and the possibility, that collected samples can be lost or misidentified. Infection and inconvenience are magnified in situations in which frequent or regular sample collection is required, such as with UVR exposure monitoring regimes and the assessment of long-term therapy. Further, the time and cost associated with these invasive test methods make it difficult to rapidly genotype and assess DNA damage for large populations of individuals. It would, therefore, be desirable to provide a non-invasive or minimally invasive skin collection methodology that may be conducted easily and rapidly in a home, clinical or cosmetic setting.

This background information is provided for the purpose of making known information believed by the applicant to be of possible relevance to the present invention. No admission is necessarily intended, nor should be construed, that any of the preceding information constitutes prior art against the present invention.

### SUMMARY OF THE INVENTION

An object of the present invention is to provide methods for assaying MC1R variants and mitochondrial markers in skin samples. In accordance with an aspect of the present invention, there is provided a diagnostic method for determining the skin state and genetic predisposition of a subject to UVR damage, comprising:
(a) assaying a skin sample from the subject for a mitochondrial DNA (mtDNA) aberration, wherein the aberration is a 3895 bp mtDNA deletion between nucleic acids 546 to 4444 of the mtDNA genome;
(b) assaying a tissue sample from the subject for one or more melanocortin 1 receptor (MC1R) variants, wherein the one or more MC1R variants are selected from the group consisting of D84E, R142H, R151C, R160H, D294H, V60L, and V92M; and
(c) determining the skin state and genetic predisposition of the subject to UVR damage based on the detection of the mtDNA aberration and MC1R variant(s) in the skin and tissue samples, respectively.

In accordance with another aspect of the invention there is provided use of the method of the invention for predicting photoaging, UVR damage or skin disease or for determining a prophylactic or therapeutic treatment for preventing or ameliorating photoaging, UVR damage or skin cancer.

In accordance with another aspect of the invention there is provided a method for monitoring a subject's response to a preventative or therapeutic treatment for photoaging, UVR damage or skin disease, comprising:
(a) assaying a first skin sample from the subject for a mitochondrial DNA (mtDNA) aberration, wherein the aberration is a 3895 bp mtDNA deletion between nucleic acids 546 to 4444 of the mtDNA genome;
(b) assaying a tissue sample from the subject for one or more melanocortin 1 receptor (MC1R) variants, wherein the one or more MC1R variants are selected from the group consisting of D84E, R142H, R151C, R160H, D294H, V60L, and V92M;
(c) determining the skin state and genetic predisposition of the subject to UVR damage based on the detection of the mtDNA aberration in the first skin sample and the one or more MC1R variants in the tissue sample;
(d)
   assaying a second skin sample from the subject for the mtDNA aberration following a prescribed period of time after preventative or therapeutic treatment for photoaging, UVR damage or stain disease;
   repeating step (d) at regular intervals over a prescribed period of time; and
   comparing the level of the mtDNA aberration between the first skin sample and the skin samples taken at regular intervals to detect changes in the mtDNA aberration, thereby monitoring the effectiveness of the treatment.

In accordance with another aspect of the invention there is provided a method of screening for an effective therapeutic or cosmeceutic agent for the treatment of photoaging, UVR damage or skin disease, comprising;
(a) assaying a first skin sample from a subject for a mitochondrial DNA (mtDNA) aberration, wherein the aberration is a 3895 bp mtDNA deletion between nucleic acids 546 to 4444 of the mtDNA genome;
(b) assaying a tissue sample from the subject for one or more melanocortin 1 receptor (MC1R) variants, wherein the one or more MC1R variants are selected from the group consisting of D84E, R142H, R151C, R160H, D294H, V60L, and V92M;
(c) determining the skin state and genetic predisposition of the subject to UVR damage based on the detection of the mtDNA aberration in the first skin sample and the one or more MC1 R variants in the tissue sample;
(d) assaying a second skin sample from the subject for the mtDNA aberration, following a prescribed period of time after treatment with the therapeutic or cosmeceutic agent; and
(e) comparing the level of the mtDNA aberration in the first skin sample and the second skin sample against a control to determine the effectiveness of the therapeutic or cosmeceutic agent.

In accordance with another aspect of the invention there is provided a diagnostic kit for determining the skin state and genetic predisposition of a subject to UVR damage, comprising:
(a) material for collecting tissue samples; and
(b) suitable primers, probes and reagents for carrying out melanocortin 1 receptor (MC1R) genotyping and the detection of a mitochondrial DNA (mtDNA) aberration;
wherein the mtDNA aberration is a 3895 bp mtDNA deletion between nucleic acids 546 to 4444 of the mtDNA genome and wherein the MC1R genotyping comprises the assaying of one or more MC1 R variants selected from the group consisting of D84E, R142H, R151C, R160H, D294H, V60L, and V92M.

### BRIEF DESCRIPTION OF THE FIGURES

These and other features of the invention will become more apparent in the following detailed description in which reference is made to the appended drawings.
Figure 1 shows real-time PCR data relating to the 3895 bp mtDNA deletion levels in skin samples collected from the nose and the heel using the method of the present invention.
Figure 2 shows real-time PCR data relating to levels of the 3895 bp mtDNA deletion in skin cells collected from various body sites using the method of the present invention.
Figure 3 shows real-time PCR data relating to levels of the 3895 bp mtDNA deletion in skin cells collected from various body sites using the method of the present invention.
Figure 4 is a gel showing the presence of amplification products present in samples collected from various non-invasive skin collection methods.
Figure 5 is a gel showing the presence of amplification products present in samples collected from various non-invasive skin collection methods.
Figures 6 to 11 show genotyping results for subjects tested for allele variants.
Figure 12 shows a gel providing results of an assay testing for the R160H allele variant.
Figure 13 shows a graph depicting the level of 3895 bp mtDNA deletion of a population categorized into age cohorts.

### DETAILED DESCRIPTION OF THE INVENTION

The present invention provides methods of predicting, diagnosing and monitoring skin states and skin diseases. The methods comprise coupling non-invasive skin sampling techniques with assays that determine mitochondrial mutations and Melanocortin 1 Receptor (MC1R) variants. In this regard, the methods provide a comprehensive tool for assessing genetic predisposition and risk factors associated with disease, ageing and ultraviolet radiation (UVR) damage. The methods also allow for the assessment of a patient's response to therapeutic agents, skin care products and treatment regimes.

### Definitions

Unless defined otherwise, all technical and scientific terms used herein have the same meaning as commonly understood by one of ordinary skill in the art to which this invention belongs.

As used herein, the term "about" refers to approximately a +/-10% variation from the stated value. It is to be understood that such a variation is always included in any given value provided herein, whether or not it is specifically referred to.

As used herein, "alleles" means one of several alternative forms of a given DNA sequence occupying a specific place on a chromosome.

As used herein, "cycle threshold" (C_{T}) is the point at which target amplification of a nucleic acid sequence rises above background, as indicated by a signal such as a fluorescence signal. The C_{T} is inversely related to the quantity of the sequence being investigated.

As used herein, "diagnostic" or "diagnosing" means using the presence or absence of a mutation or combination of mutations as a factor in disease diagnosis or management. The detection of the mutation(s) can be a step in the diagnosis of a disease.

As used herein, "deletions" means removal of a region of DNA or mtDNA from a contiguous sequence of a nucleic acid. Deletions can range in size from one base to thousands of bases or larger.

As used herein, "mitochondrial DNA" or "mtDNA" is DNA present in mitochondria.

As used herein, "mutation" encompasses any modification or change in nucleic or mitochondrial DNA from the wild type sequence, including without limitation point mutations, transitions, insertions, transversions, translocations, deletions, inversions, duplications, recombinations or combinations thereof. The modification or change of the sequence can extend from a single base change to the addition or elimination of an entire DNA fragment.

The term "sample" refers to any preparation derived from skin of a subject. For example, a sample of cells obtained using the non-invasive method described herein can be used to isolate polynucleotides, polypeptides, or mitochondrial DNA, for the methods of the present invention. Samples for the present invention, typically are taken from the dermis or epidermis of the skin. The samples are preferably taken of the skin surface using non-invasive or minimally invasive skin sampling methods discussed herein.

The term "skin" refers to the outer protective covering of the body, consisting of the corium and the epidermis, and is understood to include sweat and sebaceous glands, as well as hair follicle structures. In one embodiment, the skin is mammalian skin, preferably human.

As used herein the term "skin state" refers to the condition of the skin with respect to the amount of UVR damage accumulated due to sun exposure, tanning beds or UVR associated diseases and the like.

The terms "therapy" and "treatment," as used interchangeably herein, refer to an intervention performed with the intention of improving a subject's status. The improvement can be subjective or objective and is related to ameliorating the symptoms associated with, preventing the development of, or altering the pathology of a skin disease, disorder or state. Thus, the terms therapy and treatment are used in the broadest sense, and include the prevention (prophylaxis), moderation, reduction, and curing of a disease, disorder or state, at various stages. Preventing deterioration of a subject's status is also encompassed by the term. Subjects in need of therapy/treatment thus include those already having the disease, disorder or state as well as those prone to, or at risk of developing, the disease, disorder or state and those in whom the disease, disorder or state is to be prevented.

### Assays for Determining Skin State and Assessing Genetic Predisposition to Skin Disease

The methods of the present invention comprise coupling non-invasive skin sampling techniques with assays effective in identifying an individual's genetic predisposition to skin disease and risk factors associated with UVR exposure. The assays may be conducted alone or in combination with one another. The combined use of these assays provides a unique ability to simultaneously assess genetic risk factors and sun lifestyle habits, thereby providing a diagnostic tool for health care professionals to better monitor, advise and treat patients that are prone to or suffering from photoaging, UVR damage or a skin disease.

### Assay for Detection of Mitochondrial Mutations

Mitochondrial DNA (MtDNA) dynamics are an important diagnostic tool. Mutations in mtDNA are often preliminary indicators of developing disease and may act as biomarkers indicative of risk factors associated with disease onset. As well, as a result of the higher copy number per cell of mitochondrial DNA as compared to nuclear DNA it is a more robust target for assays relying on extremely minute quantities of nucleic acids. The methods of the present invention, therefore, couple non-invasive techniques for collecting skin samples with an assay for detecting mutations in human mitochondrial genome.

As discussed herein, measuring the level of mitochondrial DNA aberrations in a skin sample can identify the skin state of a patient with respect to cumulative sun exposure and UVR damage. Furthermore, measurement of mtDNA at regular intervals such as biweekly or monthly can provide health care professionals with a real-time, quantitative monitoring tool for comparison against treatment recommendations in order to determine their effectiveness in preventing skin damage caused by UVR.

The present invention, therefore, provides a method for determining the skin state of a patient with respect to cumulative UVR damage, comprising combining a non-invasive skin collecting technique with an assay for detecting a mitochondrial aberration which is the 3895 bp mtDNA deletion identified in PCT application no. WO/06/111029. In accordance with one embodiment of the invention, the mutation is the 3 895 bp deletion as set forth in SEQ ID NO:1. In accordance with another embodiment of the invention, the mtDNA mutation corresponds to the sequence as set forth in SEQ ID NO:2.

Exemplary methods for non-invasively collecting skin samples and assaying mitochondrial mutation are provided in the Examples section. Extraction of mtDNA from a sample may be undertaken using any suitable known method. MtDNA extraction is followed by amplification of all or a region of the mitochondrial genome, and may include sequencing of the mitochondrial genome, as is known in the art and described, for example, in Current Protocols in Molecular Biology (Ausubel et al., John Wiley & Sons, New York, 2007). Likewise, methods for detecting the presence of mutations in the mtDNA can be selected from suitable techniques known to those skilled in the art. For example, analyzing mtDNA can comprise sequencing the mtDNA, amplifying mtDNA by PCR, Southern, Northern, Western South-Western blot hybridizations, denaturing HPLC, hybridization to microarrays, biochips or gene chips, molecular marker analysis, biosensors, melting temperature profiling or a combination of any of the above.

Any suitable means to sequence mitochondrial DNA may be used. Preferably, mtDNA is amplified by PCR prior to sequencing. The method of PCR is well known in the art and may be performed as described in Mullis and Faloona, 1987, Methods Enzymol., 155: 335. PCR products can be sequenced directly or cloned into a vector which is then placed into a bacterial host. Examples of DNA sequencing methods are found in Brumley, R. L. Jr. and Smith, L.M., 1991, Rapid DNA sequencing by horizontal ultrathin gel electrophoresis, Nucleic Acids Res. 19:4121-4126 and Luckey, J.A., et al, 1993, High speed DNA sequencing by capillary gel electrophoresis, Methods Enzymol. 218: 154-172. The combined use of PCR and sequencing of mtDNA is described in Hopgood, R., et al, 1992, Strategies for automated sequencing of human mtDNA directly from PCR products, Biotechniques 13:82-92 and Tanaka, M. et al, 1996, Automated sequencing of mtDNA, Methods Enzymol. 264: 407-421.

### Melanocortin 1 Receptor (MCIR) Genotyping

The melanocortin 1 receptor is a key control point in melanogenesis and determines the amount of pigment accumulation in the skin. Given that skin pigment determines the degree of an individual's natural protection to carcinogenic UVR, MC1R genotyping can determine susceptibility to DNA damage caused by UVR exposure and assess risk factors associated with skin cancer. As such, evaluating the DNA or amino acid sequence of the Melanocortin 1 Receptor (MC1R) gene to identify whether certain variants which are associated with increased sun sensitivity and susceptibility to UVR damage, provides a valuable tool for identify individuals at greater risk for disease and who may be in need of more aggressive monitoring and treatment measures.

The methods of the present invention couple non-invasive skin collecting techniques with an assay for MC1R genotyping. DNA or RNA extracted from skin samples may be used to identify one or more MC1R variant alleles. For example, the seven allele variants D84E, R142H, R151C, R160H, D294H, V60L, and V92M associated with increased melanoma risk may be tested simultaneously. Alternatively, MC1R genotyping for the five allelic variants most commonly associated with increased risk of skin cancer, i.e. D84E, R151C, D294H, V60L, and V92M may be undertaken. The MC1R gene, including variants, is set forth in SEQ ID NO: 3. In addition, the MC1R RNA sequence is set forth in SEQ ID NO: 4.

The present invention, therefore, provides a method for determining the genetic predisposition of an individual to skin damage or cancer comprising combining a non-invasive skin collecting technique with an assay for identifying the presence of one or more MC1R variants in a skin sample, wherein the MC1R variants are selected from the group consisting of allelic variants D84E, R142H, R151C, R160H, D294H, V60L, and V92M.

Exemplary methods for non-invasively collecting skin samples and performing MC1R genotyping are provided in the Example section. One of skill in the art will appreciate that, unlike the detection of mtDNA mutations, which may fluctuate through the lifetime of an individual as a result of UVR exposure patterns, the results from MC1R genotyping will not change throughout an individual's life. Accordingly, MC1R may only be tested once during the lifetime of a patient, if so desired.

Genotyping assays such as those used for evaluating the MC1R variants will produce identical results in an individual regardless of which body tissue is samples as the sequence of the MC1R gene is inherited and does not change throughout ones lifetime. An individual skilled in the art would recognize that though the sample can be acquired from the non-invasive collection method for skin it may also be collected from any other body tissue to obtain the same genotype. Collection of suitable tissues for use in the methods of the invention would be well understood in the art and may include such nonlimiting examples as buccal tissue, muscle tissue, nerve tissue and the like. The same is not true for the mitochondrial assays which are tissue-specific somatic mutations.

Extraction of DNA or RNA from a sample may be undertaken using any suitable known method. Detection of specific alleles may be determined using such art recognized techniques as ABI's a qPCR Taqman SNP genotyping assay (https://products.appliedbiosystems.com/ab/en/US/direct/). Likewise, methods of preparing customized primers and probes are well known in the art (see, for example, Ausubel, et al., Current Protocols in Molecular Biology, John Wiley & Sons, Inc., NY).

Alternatively, allele variant assessment may be based on evaluating the amino acid sequence of the MC1R gene. Techniques for undertaking amino acid analysis are well understood in the art and may include, for example, high performance liquid chromatography (HPLC).

### Determining Skin Type and Risk of Skin Cancer

Not only can the methods of the invention be used to assess an individual for the presence of allelic variants, but may also be utilized to determine a skin type that is an approximation of the Fitzpatrick phototype of the individual. In this regard, the results of MC1R genotyping additionally allows for the grouping of individuals into specific skin types characterized by the associated risk for cancer.

These skin types are defined below:
Skin Type 1 is characterized in that the individual has two or more of the four allelic variants most associated with risk of skin cancer.
Skin Type 2 is characterized in that the individual has one of the four allelic variants most associated with risk of skin cancer.
Skin Type 3 or higher is characterized in that the individual has none of the four allelic variants most associated with risk of skin cancer.

Thus, in accordance with one embodiment, there is provided a method of identifying an individual's skin type and skin cancer risk factor on the basis of MC1R genotyping.

### Non-invasive Methods of Collecting Skin Tissues

The present invention makes use of non-invasive or minimally invasive techniques of collecting skin samples for genotyping or diagnostic tests. In the context of the present invention, "minimally invasive" refers to those techniques that result in the penetration of one or more layers of the skin, but draw little or no blood. Non-limiting examples of non-invasive or minimally-invasive techniques used for collecting skin samples include, but are not limited to, tapelift using surgical tape, Sterile swab wetted with 8% mandelic acid, Sterile swab wetted with distilled water, wax strip, cotton tip swap, scraping of skin using a sterile surgical blade, scraping of skin using a wooden scraper, sticky surface of an adhesive pad (CapSure™ Clean-up Pad, Arcturus), film from LCM MacroCap™ (Arcturus), heated film from LCM MacroCap™ (Arcturus) and employing a small gauge needle (for example, 28 gauge), to collect micro-cores of skin tissue.

The sample may be collected from the dermal or epidermal layer of the skin and may be derived from such areas of the body as, for example, the heel, nose, inner arm, ear, mouth, scalp, chest, shoulder, buttock, back, face, nape of the neck, hand and/or head. The sample can be used either directly as obtained from the source or following a pre-treatment to modify the character of the sample. Thus, the skin sample can be pre-treated prior to use, for example, with preservatives, reagents, and the like.

One skilled in the art will understand that more than one sampling technique may be employed at a single time. Furthermore, where a course of collections are required, for example, for the monitoring of a skin state over time, the same or different techniques may be used alone or together throughout the test period. In this regard, skin collections may be taken once only, or at regular intervals such as biweekly or monthly.

One of skill will also appreciate that certain collection methods yield extremely low levels of nucleic acids (approximately 0.1ng) which would not be useful for an assay targeting nuclear DNA; however, mitochondrial DNA targets that are in much greater abundance (approximately 1000 fold greater) would be uniquely suited to a collection method with such extremely low yields. The examples below show that skin cells collected via the non-invasive methods provide sufficient mtDNA for obtaining results comparable to mtDNA obtained via previously used skin collection methodologies.

A non-invasive collection technique may involve the use of a sterile swab, such as those used in the collection of buccal cells or cotton-tip swabs. The sterile swab is removed from its packaging and is rubbed on a skin site of interest. Preferably, the site is swabbed approximately 15 times in order to ensure that a sufficient number of skin cells are collected for genotyping or diagnostic purposes. Although the present invention is described below with reference to a specific example, the method may also be used to collect skin samples for the diagnosis or characterization of disease, aging, or exposure to ultraviolet radiation, and the identification of mutations associated therewith.

Following the swabbing of the skin, the swab is deposited into a sterile tube. Buffer may be added to the tube as necessary in order to maintain the integrity of the genetic material (i.e. DNA) contained therein. The DNA is then extracted utilizing well known methods in the art.

Alternatively, a minimally invasive technique may employ a very small gauge needle (28 or 29 gauge) is used to collect skin cells for the purpose of genetic investigation. Here, skin cells are collected from the dermis and epidermis of a subject by piercing through a tented layer of the skin such that little or no blood is drawn, but a microscopic amount of dermal and epidermal tissue is adhered to the inner core of the needle. The skin may be tented by raising the skin using, for example, fingers, tweezers, or other forms of clamp. The skin material is contained in the needle until it is extracted for further processing (ie. DNA extraction). To express the skin sample from the needle, phosphate buffered saline is deposited into the column of the needle and then forced through with the plunger into a sterile tube. DNA is extracted utilizing well known methods in the art. As illustrated by example below, this minimally invasive method for the collection of a skin sample yields sufficient DNA or mtDNA for the assessment of DNA or mtDNA damage, for example, caused by UV radiation. This method of obtaining skin samples is safe and painless. Further, as illustrated below, allows for sufficient DNA or mtDNA to be collected for conducting accurate assays.

### Diagnosing and Monitoring Skin States and Detecting Genetic Risk Factors Associated with Disease

As described herein, the assays of the invention may be used alone, for example to assess skin state or genetic risk, or in combination to evaluate both genetic predisposition to skin disease and identification of risk factors associated with an individual's particular lifestyle. As discussed below, the identification and monitoring of these factors are important in the determination of effective preventative and treatment measures against UVR damage, photoaging and skin disease.

### Diagnosing and Monitoring Skin States

Many individuals who use skin care products such as sunscreens and sunblocks regularly use the products improperly, failing to apply them in sufficient quantity or to reapply at the recommended intervals, creating a false sense of protection that can lead to increased exposure to UVR. Furthermore, until more recently, sunscreens and sunblocks regularly applied by individuals during sun exposure generally protected against the mutagenizing effects of UVB, but failed to contain agents directed at the harmful effects of UVA radiation.

These factors among others necessitate the availability of a tool to determine both initial skin state with respect to UVR damage and, through a course of studies, monitor the success and appropriateness of preventative measures and therapies being advised to prevent further UVR damage to the skin. Measuring the level of mitochondrial DNA deletions in the skin of a patient by non-invasively collecting skin samples at regular intervals such as biweekly or monthly (or any other suitable interval) can provide health care professionals with a real-time, quantitative monitoring tool to compare against treatment recommendations to determine their effectiveness in preventing skin damage caused by UVR. This collection methodology permits regular sampling without any side effect or discomfort and can be performed at home, if desired. Surprisingly, techniques for collecting skin samples such as swabbing are uniquely suited to the detection of mtDNA mutations, as the extremely low yields of DNA provided by swabbing are suffice for undertaking such analysis.

Turning now to the examples, in one embodiment of the invention the method is used to collect skin cells for the quantification of biomarkers associated with damage caused by UV radiation (see Example 1). Specifically, the method of the present invention was used to collect skin samples for testing for deletions in the human mitochondrial genome, namely the 3895 bp mtDNA deletion described above. The example shows that skin cells collected via the non-invasive method of the present invention provides sufficient mtDNA for obtaining results comparable to mtDNA obtained via previous skin collection methodologies.

One of ordinary skill in the art will understand that the non-invasive collection of DNA samples from the epidermal or dermal layers of human skin for quantification of a mitochondrial DNA target provides a means for a health care worker to monitor the effectiveness of treatment regimes and lifestyle recommendations for the prevention of UVR associated skin and DNA damage with the intent to prevent such consequences as photoaging and skin cancer.

One of ordinary skill will also appreciate the utility of mtDNA analysis for use by health care providers in identifying poor sun habits or ineffective protection methods. Such utility is demonstrated in Example 6, where according to one embodiment of the invention, the results of sunscreen use was analysed for two individuals of similar age and skin tone to assess the effectiveness of sun protection regimes.

### Determination of Photoage

An additional diagnostic method of the invention that makes use of a minimally invasive sampling procedure coupled with the identification of a mitochondrial marker is the determination of photoage. As described in the Example section, the purpose of this method is to evaluate an individual's UVR exposure by sampling the individual's skin tissue on the face or head using a minimally invasive skin collecting technique and measuring the amount of a mitochondrial target associated with UVR damage such as mtDNA deletion. This result is then compared against a database of results achieved by the same method and the individual is classified into an age cohort based upon the level of mtDNA deletion detected. For assignment of a photoage, the results of an individual's mtDNA analysis is compared against their chronological age to determine if they have higher or lower photodamage than other individuals in their age group.

Thus, by utilizing minimally invasive techniques to collect micro-cores of skin tissue for analysis of mitochondrial DNA markers, health care provides can identify an individual's photoage and determine whether their photoage is consistent or inconsistent with their chronological age. Determination of photoage thereby provides a means for health care professionals to more appropriately prescribe preventative and treatment measures to combat photoaging, UVR damage or skin disease.

### Determining Genetic Predisposition to Disease

In order to fully evaluate an individual's risk of photoaging and their risk of skin cancer it is imperative that health care providers are provided with as much information as possible to understand and communicate their patient's risk factors. The utilization of MC1R genotyping not only contributes to an individual's susceptibility to DNA damage caused by UVR exposure and risk of developing skin cancer, it provides a valuable tool to identify patients with greater risk who are potentially in need of more aggressive monitoring and treatment measures.

The present invention therefore provides a non-invasive method for the collection .of DNA samples for MC1R genotyping. In accordance with one embodiment of the invention, DNA extracted from cells collected by the method of the present invention may be used to identify one or more allele variants associated with increased melanoma risk. The method may be used to assess an individual for the presence of these variants and to additionally determine a skin type of the individual. The results of MC1R genotyping therefore allows for the grouping of individuals into specific skin types characterized by the associated risk for cancer.

### Coupled Analysis

The combined use of MC1R genotyping and mtDNA analysis provides a tool for health care professionals to monitor, advise and better treat patients by considering both genetic predisposition and the end results of their sun lifestyle habits. This combined use of assays provides a unique ability to simultaneously assess risk factors as well as consequences. Importantly the MC1R test may be temporally removed from repeated testing with the non-invasive collection for mtDNA testing and still be used in tandem. As discussed above, this is possible because the information obtained from the MC1R test will not change throughout an individual's life though the DNA damage levels elucidated by measuring mitochondrial DNA deletion will fluctuate as a result of UVR exposure patterns.

Noteworthy is the fact that skin damage caused by UVR is a multifactorial process. Accordingly, it cannot be assumed that an individual with MC1R variants will always have higher damage than an individual without, nor will it be the case that an individual who reports regular sunscreen use will necessarily have lower damage than one who reports no sunscreen use. Thus, the coupling of MC1R genotyping with mtDNA analysis provides health care workers. with the unique insight they require to make more informed recommendations to those in their care.

### Evaluation of Therapeutic Agents and Skin Care Products

The method of the present invention may also be used for widespread skin screening for both medical and cosmeceutical purposes. The method of the present invention may be used for genotyping and/or to measure various biomarkers associated with skin cancer (both non-melanoma skin cancer and melanoma). The ability to assess the level of DNA damage in an individual's skin due to UV radiation at any time point and from any external anatomical location provides the foundation for a unique and informative screening test for skin health and to assess the safety and efficacy of existing and new therapeutic agents, skin care products and skin care regimes. Furthermore, by identifying the specific genetic changes underlying a subject's skin disease or state, it may be readily determined whether and to what extent a patient will respond to a particular therapeutic agent, skin care product or regime.

### Kits

The collection materials used in the method of the present invention may be packaged, depending on the desired application, into a consumer kit or a medical kit to be used in a clinical environment. Such kits could not only include one or more sampling means, such as sterile swabs, cotton tip swabs or needles, but other materials necessary for genotyping and/or the identification of mtDNA mutations.

The kits can optionally include reagents required to conduct a diagnostic assay, such as buffers, salts, detection reagents, and the like. Other components, such as buffers and solutions for the isolation and/or treatment of a test sample, may also be included in the kit. One or more of the components of the kit may be lyophilised and the kit may further comprise reagents suitable for the reconstitution of the lyophilised components.

Where appropriate, the kit may also contain reaction vessels, mixing vessels and other components that facilitate the preparation of the test sample. The kit may also optionally include instructions for use, which may be provided in paper form or in computer-readable form, such as a disc, CD, DVD or the like.

To gain a better understanding of the invention described herein, the following examples are set forth. It will be understood that These examples are intended to describe illustrative embodiment of the invention.

### EXAMPLES

### Example 1: Analysis of 3895 bp human mtDNA deletion

The method of the present invention was used to analyze the 3895 bp mtDNA deletion identified in PCT application no.WO/06/111029. Collection and extraction of the mtDNA was conducted as provided below.
1. Skin samples were collected by swabbing a skin site approximately 15 times with a sterile swab. Skin samples were collected from heel (n= 41), nose (n= 43), inner arm (n= 20), ear (n= 5), shoulder (n= 5), buttock (n= 5), and back (n= 5).
2. mtDNA was extracted using a commercially available kit (QiaAMP™ DNA Micro Kit, product no. 56304, *Qiagen,* Maryland USA) according to the manufacturer's protocol.
3. Double stranded DNA was quantified using the HS-DNA Quant-it™ dsDNA HS Assay Kit (product no. Q32851, *Invitrogen,* California USA) on the Qubit™ Fluorometer (product no. Q32857), *Invitrogen,* California USA).
4. The level of the 3895 bp deletion was then quantified by real-time PCR (rt-PCR) using the iQ Sybr Green Supermix™ (product no. 170-8882, *Bio-Rad,* California USA) and the following primers:
   Forward 5'-CTGCTAACGCCATACCCCGAAAATGTTG-3' (SEQ ID NO: 5);
   Reverse 5'-GAAGGATTATGGATGCGGTTGCTTGCGTGAG -3' (SEQ ID NO: 6).

In this example, the pair of amplification primers are used to amplify a target region indicative of the presence of the 3895 bp deletion. The forward primer overlaps a spliced region of mtDNA after deletion of the 3895 bp sequence has occurred (ie. a splice at a position between 547 and 4443 of the mtDNA genome). Therefore, extension of the overlapping primer to create the correct size amplification product can only occur if the 3 895 bp section is deleted.

In the step of quantifying the 3895 bp deletion, the RT-PCR reaction was set up as follows:
12.5ul of iQ Sybr Green Supermix™;
350nmol forward primer (SEQ ID NO: 5);
350nmol reverse primer (SEQ ID NO: 6);
5ul of template (approximately 0.5ng dsDNA);
water to 25ul;

Cycling parameters:
Step 1. 95°C for 3 minutes;
Step 2. 95°C for 30 seconds;
Step 3.67.5°C for 30 seconds;
Step 4.72°C for 30 seconds;
Step 5. Plate Read
45 cycles of steps 2-5
Melting Curve 55-110°C reading every 3 seconds at 1°C intervals
Hold at 10°C for 10 minutes.

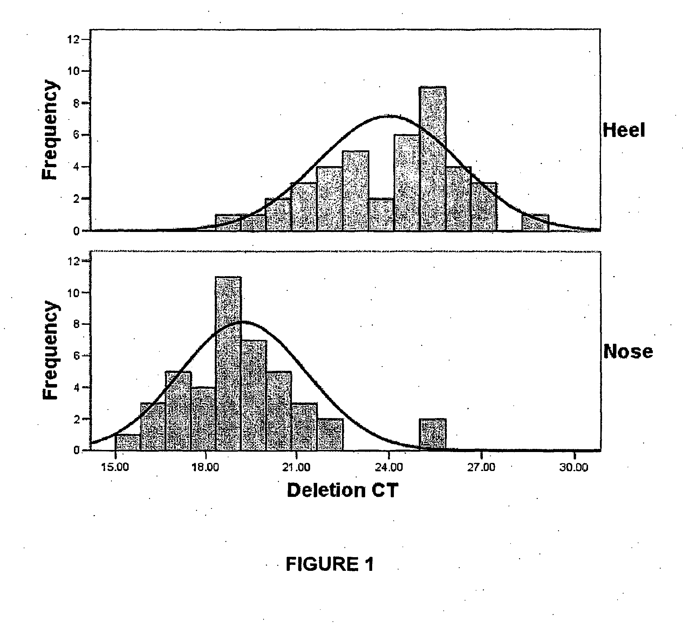

The results of these assays are shown in Figures 1 to 3 and demonstrate a clear distinction between skin swabs taken from areas rarely exposed to sunlight/UV radiation (ie. heel and buttocks) and those usually exposed (ie. nose and ear). Levels of the 3895 bp deletion are significantly elevated in areas receiving a higher level of UV radiation such as the nose and shoulder when compared to areas generally protected from UV radiation such as the heel and the buttocks.

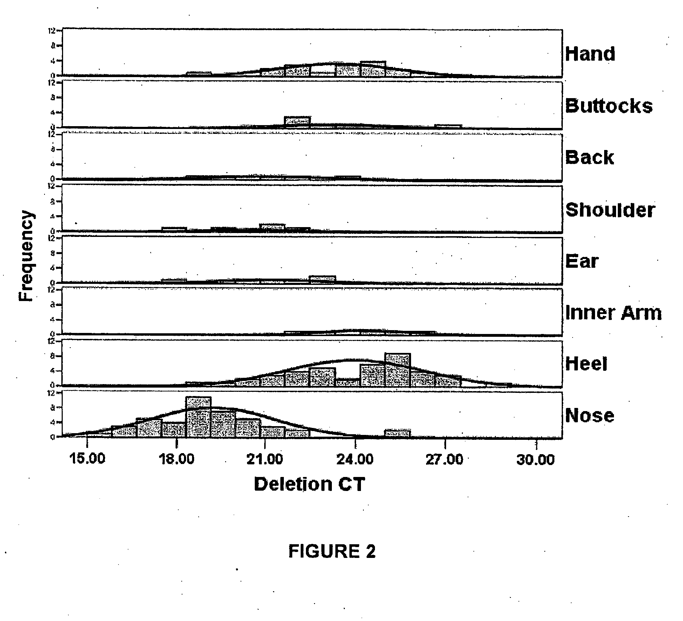

As shown in Figures 1 and 2, the real time PCR cycle thresholds (C_{T}) for the 3895 bp deletion indicate that there is a higher incidence of the deletion in skin sites usually (nose or ear) or occasionally (shoulder or back) exposed to UV radiation compared to those sites that are rarely exposed (heel or buttocks).

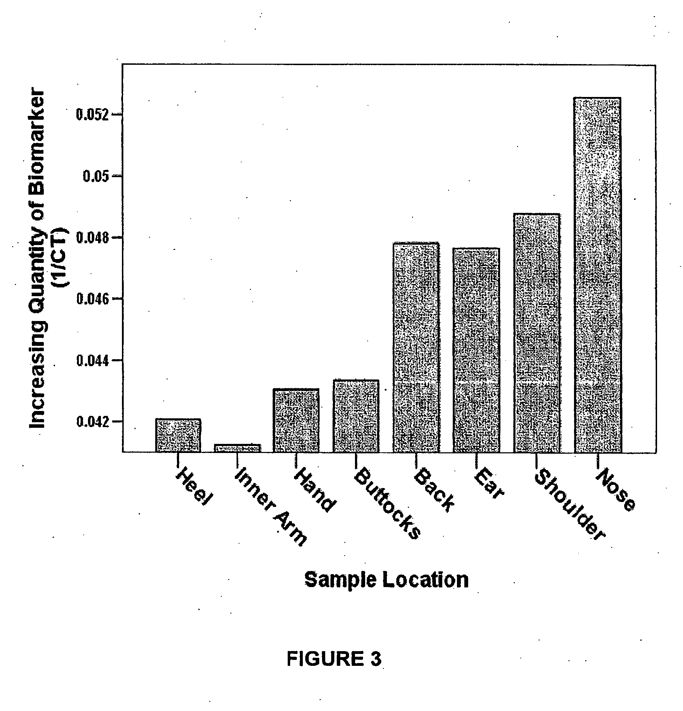

Figure 3 shows that mtDNA collected from skin cells obtained from sites that are usually exposed to UV radiation (e.g. nose or ears) are characterized by increased levels of the 3895 bp deletion marker than mtDNA collected from skin cells obtained from sites rarely exposed to UV radiation (e.g. heel or inner arm).

These results also show the effectiveness of collecting skin samples in accordance with the present invention, in order to obtain sufficient mtDNA to conduct the assays. As such, the non-invasive skin collection methods of the present invention are similarly effective for obtaining mtDNA for analysis as invasive methodologies, for example, the methods used in the Applicant's PCT publication no.WO/06/111029.

### Example 2: Comparison of Skin Collection Methods

Five different non-invasive skin collection methodologies were tested in order to identify which, if any, would yield sufficient quantity and quality of nucleic acids for molecular analyses such as quantitative real-time PCR. The five methods tested were:
- Tapelift using surgical tape;
- Biore® adhesive strip;
- Sterile swab wetted with 8% mandelic acid;
- Sterile swab wetted with distilled water; and
- Wax strip.

The tapelift, Biore strip and wax strip were applied to the surface of the skin following the application of 70% isopropanol to sterilize the area. Firm pressure was applied and then the tape or strip was removed quickly. The swabs were first deposited in a sterile solution of either 8% mandelic acid, or distilled water and then rubbed firmly on the skin site of interest after the skin had been cleaned with 70% isopropanol.

Following the collection of skin cells from 3 individuals each collection medium was deposited into 200ul phosphate buffered saline solution (PBS) and incubated overnight at 56°C.

All of the samples were then subjected to nucleic acid extraction using the Qiagen's QiaAMP™ DNA Mini Kit , buccal swab protocol (product no. 51304). The purified samples were quantified using the NanoDrop™ ND-1000 Spectrophotometer to determine if the extraction procedure was successful.

**Table 1: Determination of Quantity of DNA Extracted from Skin Collected by Five Non-invasive Methods**

| **Sample ID** | **DNA concentration ng/uL** | **A260nm** | **DNA Parity 260:280 Ratio** |
|---|---|---|---|
| 1 biore | -0.42 | -0.008 | 0.48 |
| 1 swab with water | 5.03 | 0.101 | 1.6 |
| 1 swab with mandelic acid | 5.05 | 0.101 | 1.74 |
| 1 surgical tape | 2.22 | 0.044 | 4.4 |
| 1 wax | 3.52 | 0.07 | 1.69 |
| 2 biore | -0.02 | 0 | -0.09 |
| 2 swab with water | 2.74 | 0.055 | 1.97 |
| 2 swab with mandelic acid | 4.9 | 0.098 | 1.82 |
| 2 surgical tape | . 2.99 | 0.06 | 1.78 |
| 2 wax | 2.17 | 0.043 | 1.84 |
| 3 biore | -0.26 | -0.005 | 0.24 |
| 3 swab with water | 3.26 | 0.065 | 3.15 |
| 3 swab with mandelic acid | 2.71 | 0.054 | 12.1 |
| 3 surgical tape | 2.45 | 0.049 | 4.26 |
| 3 wax | 2.67 | 0.053 | 4.25 |

When considering both nucleic acid concentration as well as the purity of the sample, the most consistent results were achieved for the swab samples using either water or mandelic acid as a wetting agent, or the wax samples.

Next, a PCR was performed on all samples to determine if amplification inhibitors were present or significant degradation of the sample had occurred during processing. Samples were amplified according to the following conditions:

**Table 2: PCR Conditions for Sample Amplification**

| **Reagent** | **Final Concentration in Reaction** |
|---|---|
| 10X reaction Buffer | 1X |
| dNTPs | 0.4 mM each |
| BSA | 1X |
| 12s primer (forward) | 0.4 uM |
| 12s primer (reverse) | 0.4 uM |
| Taq LA (Takara p/n RR002B | 1.25 Units |
| Template | 5 ul (of above concentration) |
| Water | To 25ul |

The primers used were mitochondrial DNA primers having the sequences provided below: 12s primer sequence forward 5'-CGTTCCAGTGAGTTCACCCTC-3' (SEQ ID NO: 7) 12s primer sequence reverse R 5'-CACTCTTTACGCCGGCTTCTATT-3' (SEQ ID NO: 8)

The amplification reactions were cycled on a DNA Engine Tetrad (Bio-Rad) according to the following protocol:
1. 94°C for 2 minutes
2. 94°C for 30 seconds
3. 64°C for 30 seconds
4. 72°C for 30 seconds
5. Repeat steps 2-4 39 times
6. 4°C HOLD

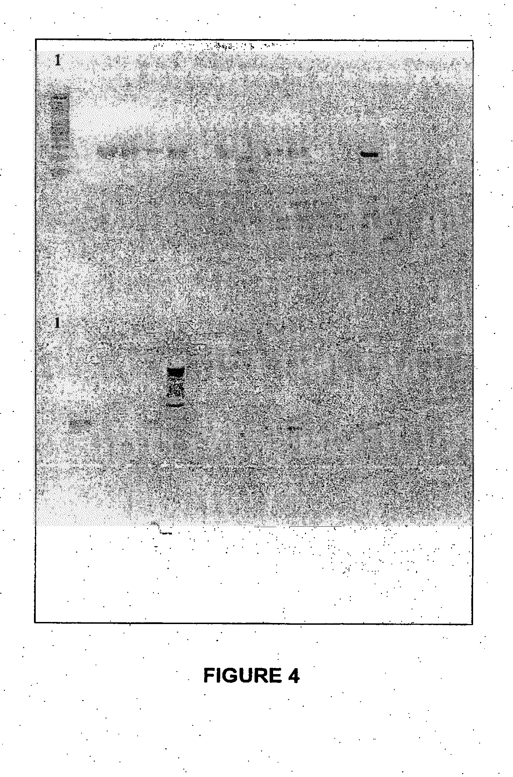

Amplification products were then electrophoresed on a 2% agarose gel and stained with ethidium bromide. The amplification results are provided in Figure 4, where the top half of the gel contains:
Lane 1 500ng 100bp GeneRuler SM0323 (Fermentas)
Lane 2 Biore from Individual 1
Lane 3 Swab with water from Individual 1 .
Lane 4 Swab with mandelic acid from Individual 1
Lane 5 Surgical tape from Individual 1
Lane 6 Wax from Individual 1
Lane 7 Biore from Individual 2
Lane 8 Swab with water from Individual 2
Lane 9 Swab with mandelic acid from Individual 2
Lane 10 Surgical tape from Individual 2
Lane 11 Wax from Individual 2
Lane 12 empty ,
Lane 13 Negative amplification control
Lane 14 Positive amplification control
Lanes 15-18 empty

And where the bottom half of the gel contains:
Lane 1 Biore from Individual 3
Lane 2 Swab with water from Individual 3
Lane 3 Swab with mandelic acid from Individual 3
Lane 4 Surgical tape from Individual 3
Lane 5 Wax from Individual 3
Lane 6 500ng 100bp GeneRuler SM0323 (Fermentas)
Lane 7 Biore extract negative control
Lane 8 Swab with water extract negative control
Lane 9 Swab with mandelic acid extract negative control
Lane 10 Surgical tape extract negative control
Lane 11 Wax extract negative control
Lane 12 empty
Lane 13 Negative amplification control (duplicate loading to Lane 13 above)
Lane 14 Positive amplification control (duplicate loading to Lane 14 above)
Lane 15-18 empty

### Results

No mtDNA was amplified from mtDNA collected from skin cells harvested using the Biore strips. The swabs for both the water and the mandelic acid amplified, though the water swab amplified more brightly. The surgical tape amplified sporadically. The wax amplified brightly however the extract negative control in Lane 11 of the bottom half of the gel was contaminated likely as a result of the non-sterile nature or handling difficulties associated with the wax.

With all factors considered this example demonstrated that the use of a sterile swab is the preferred method of collection of a non-invasive skin sample. The swab can be dry or wetted with various liquids to facilitate collection or buffering of the sample.

### Example 3: Comparison of Additional Skin Collection Methods

In this example five additional methods for the non-invasive collection of skin samples were tested in order to identify which, if any, would yield sufficient quantity and quality of nucleic acids for molecular analyses such as quantitative real-time PCR.

From a single individual, skin samples were collected twice using the following methods:
- scraping of skin using a sterile surgical blade
- scraping of skin using a wooden scraper
- sticky surface of an adhesive pad (CapSure™ Clean-up Pad, Arcturus)
- film from LCM MacroCap™ (Arcturus)
- heated film from LCM MacroCap™ (Arcturus)

The skin was first prepared by cleansing with a 70% isopropanol wipe. The wooden scraper and the surgical blade were passed firmly over the skin surface to remove skin cells and then deposited into a centrifuge tube. The adhesive pad and films were pressed firmly against the skin without rubbing to collect skin cells.

The multiple collections were processed using two different nucleic acid extraction methods. The first set was extracted using a proteinase K digestion as is well known in the art while the second set was extracted using the QiaAMP DNA Mini Kit (Qiagen 51304).

The samples processed with the Qiagen kit were then quantified using the NanoDrop ND-1000 Spectrophotometer. Those in the PK digestion set were not as they were not cleaned up enough to facilitate this type of quantification.

**Table 3: Determination of Quantity of DNA Extracted from Skin Collected by Further Collection Methods (Qiagen extracted DNA)**

| **Sample ID** | **DNA concentration ng/uL** | **A260nm** | **DNA Parity 260:280 Ratio** |
|---|---|---|---|
| AE Buffer | -1.09 | -0.022 | 1.6 |
| Woodscrape | 2.06 | 0.041 | 0.95 |
| Capsure | 5.59 | 0.112 | 1.29 |
| Blade | 2.32 | 0.046 | 1.41 |
| Blade -ve control | 1.19 | 0.024 | 0.8 |
| Capsure -ve control | 2.47 | 0.049 | 1.2 |
| Woodscrape -ve control | 2.92 | 0.058 | 1.35 |
| QIaGEN kIT -ve control | 2.4 | 0.048 | 1.83 |

Samples were amplified according to the protocol provided in example 2.

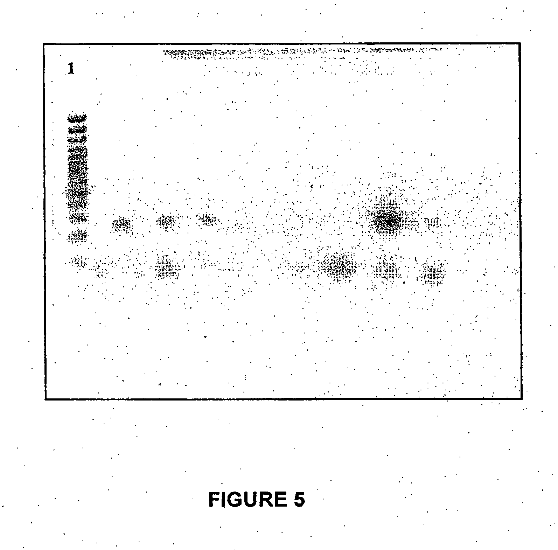

Amplification products were then electrophoresed on a 2% agarose gel and stained with ethidium bromide. Results are shown in Figure 5, where the gel contains:
Lane 1 500ng of 100bp GeneRuler (SM0323)
Lane 2 Negative Amplification control
Lane 3 Positive amplification control
Lane 4 PK buffer wood scrape
Lane 5 PK buffer surgical blade scrape
Lane 6 PK buffer CapSure pad
Lane 7 PK buffer MacroCap
Lane 8 PK buffer MacroCap heated
Lane 9 PK buffer wood scrape negative extraction control
Lane 10 PK buffer surgical blade scrape negative extraction control
Lane 11 PK buffer CapSure negative extraction control
Lane 12 PK buffer MacroCap negative extraction control
Lane 13 PK buffer MacroCap heated negative extraction control
Lane 14 QiaAMP surgical blade scrape
Lane 15 QiaAMP CapSure pad
Lane 16 QiaAMP wood scrape
Lane 17 QiaAMP surgical blade scrape negative extraction control
Lane 18 QiaAMP CapSure pad negative extraction control
Lane 19 QiaAMP wood scrape negative extraction control
Lane 20 QiaAMP reagent negative control

The surgical blade scrape and the MacroCap™ were amplified using the PK buffer, while the CapSure™ pad amplified well using the QiaAMP™ kit. When compared to skin swabbing, the amount of mtDNA collected and the amount of amplified product obtained using the methods tested in this example were not found to be as effective.

### Example 4: Collection of Skin Samples using Needle

Needles were used to collect skin samples from 5 different body sites of 9 individuals. The body sites mcluded the eyebrow, earlobe, nape of the neck, hand, and heel.

Using a needle as described above, the skin was pinched or tented between the thumb and forefinger of the sample collector's hand The needle was passed through the skin, drawing little or no blood. The skin sample was extracted from the needle by depositing phosphate buffered saline into the column of the needle and then forcing the sample from the needle with a plunger into a sterile tube. DNA was then extracted from this volume containing the skin tissue using the QiaAMP™ DNA Mini Kit™ (Qiagen product no. 51304).

The samples were then amplified in order to identify the 3895bp mtDNA deletion. The reaction conditions and cycle parameters for this example were the same as for example 1 provided above. The results are presented in Table 4

**Table 4: Results for Skin Samples collected via Needle**

| **Sample** | **Cycle Threshold C(t)** | **Sample** | **Cycle Threshold C(t)** |
|---|---|---|---|
| Subject 1 left eyebrow | 25.82 | Subject 6 left eyebrow | 22.15 |
| Subject 1 left earlobe | 25.98 | Subject 6 left earlobe | 19.87 |
| Subject 1 neck | 26.26 | Subject 6 neck | 25.87 |
| Subject 1 nght hand | 26.73 | Subject 6 right hand | 27.91 |
| Subject 1 right heel | 27.93 | Subject 7 left eyebrow | 29.89 |
| Subject 2 left eyebrow | 30.85 | Subject 7 left earlobe | 19.18 |
| Subject 2 left earlobe | 25.6 | Subject 7 neck | 25.49 |
| Subject 2 neck | 23.92 | Subject 7 right hand | 23.41 |
| Subject 2 nght hand | 27.01 | Subject 7 nght heel | 27.05 |
| Subject 2 right heel | 35.55 | Subject 8 left eyebrow | 21.82 |
| Subject 3 left eyebrow | 29.64 | Subject 8 left earlobe | 21.32 |
| Subject 3 left earlobe | 23.51 | Subject 8 neck | 23.51 |
| Subject 3 neck | 24.52 | Subject 8 right hand | 16.35 |
| Subject 3 right hand | 22.47 | Subject 8 right heel | 20.57 |
| Subject 4 left eyebrow | 24.23 | Subject 9 left eyebrow | 25.09 |
| Subject 4 left earlobe | 23.64 | Subject 9 left earlobe | 26.76 |
| Subject 4 neck | 24.96 | Subject 9 neck | 27.74 |
| Subject 4 nght hand | 25.93 | Subject 9 right hand | 25.24 |
| Subject 4 right heel | 23.58 | Subject 9 right heel | 22.39 |
| Subject 5 left eyebrow | 22.35 | | |
| Subject 5 left earlobe | 24.39 | | |
| Subject 5 neck | 22.06 | | |
| Subject 5 right hand | 22.04 | | |
| Subject 5 right heel | 22.6 | | |

It is clear that the matenal obtained through this collection method is sufficient for molecular analyses such as real-time PCR Specifically, the amplification product indicative of the 3 895 bp mtDNA deletion has been detected and quantified as evidenced by Table 4. Therefore, the collection of skin samples via the needle collection method yields sufficient DNA for use in an assay of this kind

### Example 5: Detection of MC1R Variants

Buccal samples were collected from three subjects. Each subject rinsed their mouth with tap or bottled water and expelled the water. A cotton swab was removed from its protective packaging and grasped by the handle While holding the handle of the cotton swab, the cotton tip was placed within the subject's mouth and the cotton tip was rubbed on the inside of the cheeks for 30 seconds. The swabs were placed in sterile tubes and sealed for further processing.

Detection of specific alleles was determined using ABI's a qPCR Taqman SNP genotyping assay. Each subject was tested for the seven allele variants D84E, R142H, R151C, R160H, D294H, V60L, and V92M.

The genotypes for D84E, R142H, R151C, V60L, and V92M were identified using ABI assays (https://products appliedbiosystems.com/ab/en/US/direct/) as follows
V60L-Assay #C751905410
D84E-Assay #C751905520
V92M-Assay #C203340520
R142H-Assay #C2754163410
R151C-Assay #C203340420

Customized primers and probes, as listed below, were used for the detection of the D294H allele variants.
Forward Primer MC1R-294: CGCCCTCATCATCTGCAATG SEQ ID NO: 9;
Reverse Primer MC1R-294: GGCTGTGGAAGGCGTAGAT SEQ ID NO: 10;
Probe 1MC1R-294V1VIC: CCATCATCGACCCCCT SEQ ID NO:11;
Probe 2 MCIR-294M1 FAM: CCATCATCCACCCCCT SEQ ID NO: 12.

The reactions conditions used for all assays were as outlined in the Custom Taqman™ SNP Genotyping Assays Protocol.

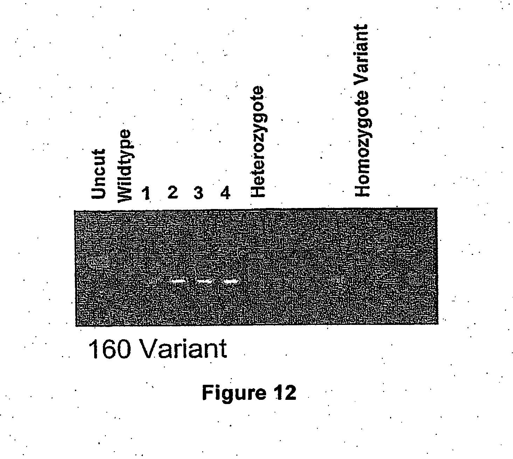

The R160H allele variant was tested for using a Sac II digest. Primers were used to amplify a target region of DNA in the MC1R gene. The amplification product was then subjected to a Sac II digest. Lanes 1 to 4 of the gel (see Figure 12) show the results for the three tested subjects and the control sample. All four of these samples were homozygote wildtype. The size of the amplification product indicative of a homozygote wildtype for this allelic site is 436 bp, which after SAC II digestion yields a product of 327 bp. In homozygote variants no digestion occurs and so all the PCR amplification product remains at a size of 436 bp.

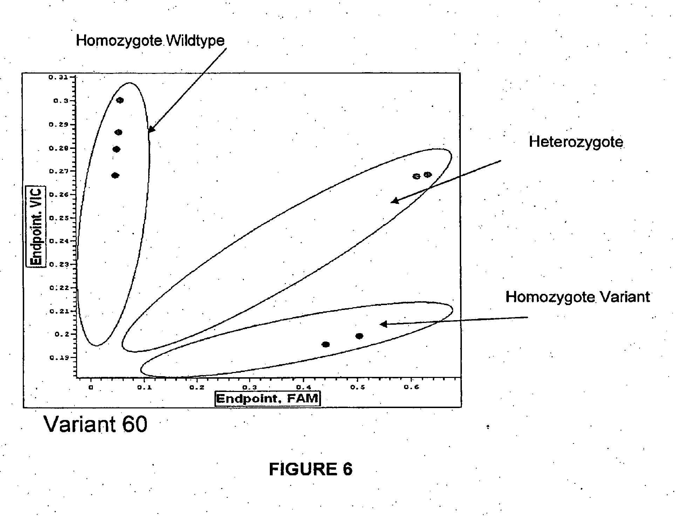

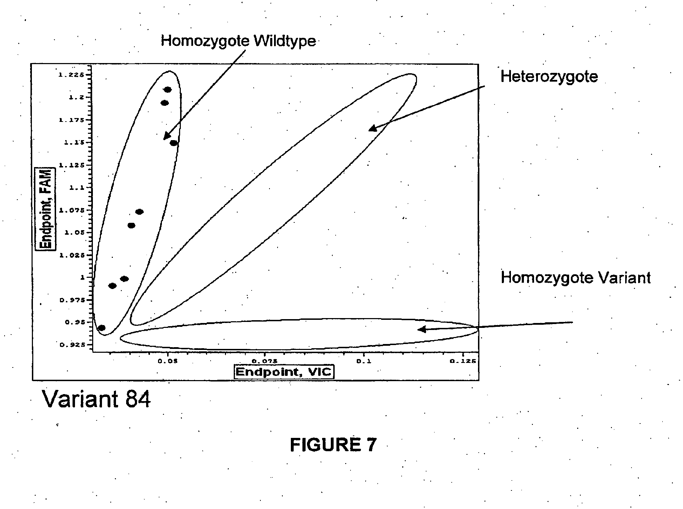

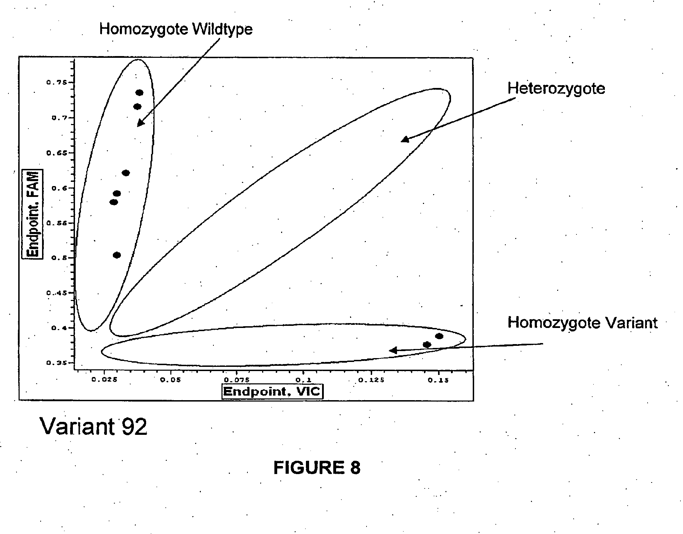

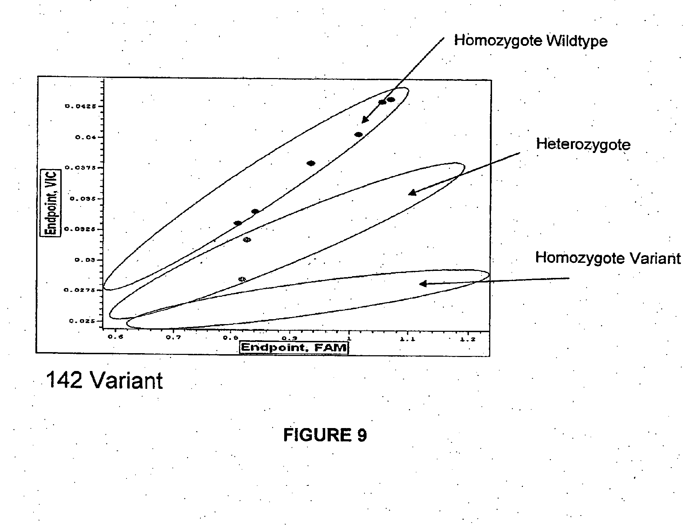

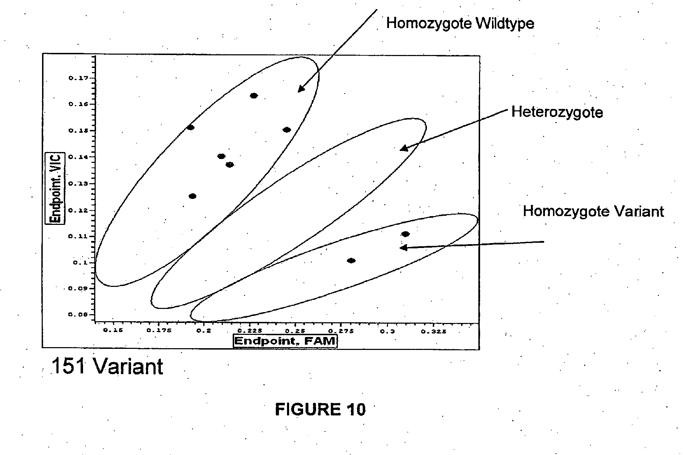

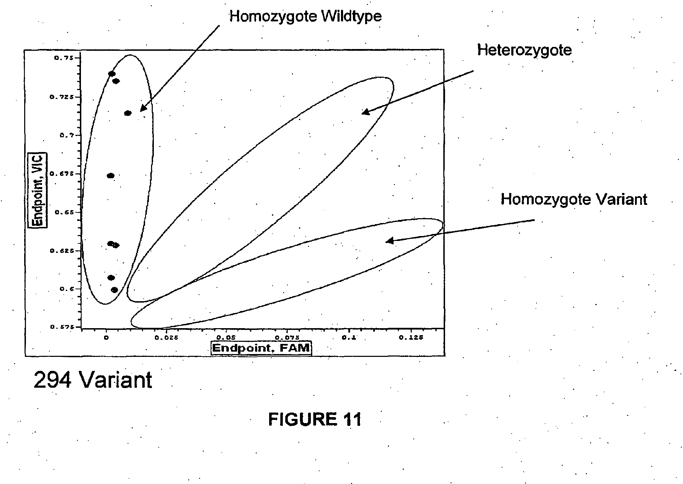

The results of the tests are shown in Figures 6 to 12. Two tests were run for each individual, and a control sample was also tested twice. The control sample genotype was a heterozygote for V60L, homozygote variant for V92M and wildtype for the remaining alleles. The first individual tested was determined to have Type 3 skin (no MC1R variants found). The second individual tested was determined to have Type 3 skin (no MC1R variants found). The third individual tested was determined to have Type 2 skin (homozygote for variants V60L and R151C and heterozygote for R142H).

It is clear that the material obtained through this collection method is sufficient for MC1R genotyping. Specifically, the collection of DNA samples via the buccal swab collection method yields sufficient DNA for use in an assay of this kind.

### Example 6: Analysis of the Effectiveness of Sunscreen Regimes

### Background:

UVB is the spectrum of UVR that causes erythema (sunburn), the visual cue that overexposure to UV has occurred. The frequency and severity of erythema is often used as a measure of success of an individual's sun protection habits. However, it is now known that UVA contributes to skin and DNA damage but it was not until recently that sunscreens and sunblocks contained agents directed at blocking UVA. Thus a scenario is created whereby an individual using sunscreen was protecting themselves against the erythema inducing effects of UVB but perhaps suffering even more prolonged exposure than they would have otherwise to the mutagenizing effects of UVA. An individual in this situation would self-report that they use sunscreens and a health care provider would mistakenly assess that measures were being taken to prevent UVR damage when in fact the patient was not being protected from UVA and ultimately risks could still be very high.

As carried out in Example 1, measuring the level of the 3895bp mitochondrial DNA deletion in the epidermis collected with a cotton swab at regular intervals such as biweekly or monthly can provide health care providers with a real-time, quantitative monitoring tool to compare against treatment recommendations to determine their effectiveness in preventing skin damage caused by UVR.

This collection methodology permits regular sampling without any side effect or discomfort. The swab yields extremely low levels of nucleic acids (approximately 0.1ng) which would not be useful for an assay targeting nuclear DNA however mitochondrial DNA targets such as the 3895bp deletion are in much greater abundance (approximately 1000 fold greater) and are uniquely suited to a collection method with such extremely low yields.

### Study:

The utility of mtDNA deletion analysis in identifying poor sun habits or ineffective protection methods is demonstrated by the results of two individuals of similar age and skin tone. Patient 202 did not use sunscreens at all while Patient 225 used sunscreens effectively. Their levels of damage reflect this behaviour with Patient 202 having approximately a 10 fold greater quantity of the 3895bp deletion indicating high levels of DNA damage from UVR exposure than Patient 225 (where a 3 CT difference is equivalent to a 10 fold difference in target quantity). Though phenotypes of these two individuals indicate that risk for UVR associated skin damage would be similar or equivalent, the 3895 deletion coupled with the non-invasive swab collection is able to demonstrate that Patient 202 is at greater risk, presumably due to behavioural differences such as sunscreen use.

**Table 5: Patient Profile and Risk Factors for UVR Damage**

| PATIENT | CT (3895 bp deletion) | AGE | SEX | Hair | Skin | Effective Sunscreen use |
|---|---|---|---|---|---|---|
| 202 | 23.16 | 25 | Male | Black | Dark Brown | No |
| 225 | 26.06 | 28 | Female | Brown | Light Beige | Yes |

### Example 7: Analysis of MC1R Variants and mtDNA Deletion to Predict Effectiveness of Sun Care Regimes

The combined utility of monitoring both the DNA damage with the UVR associated 3895 biomarker as a proxy for estimating the effectiveness of sun care regimes, as well as the determination of *MC1R* variant status is demonstrated below where two patients having similar age, skin colour, and sunscreen use have different levels of DNA damage presumably as a byproduct of different MC1R genotypes. Patient 300 has no variants associated with an increased susceptibility to the damaging effects of UVR on the skin while Patient 303 has one variant at amino acid position 92. Even though phenotypically the patients are very similar and would score similarly on the Fitzpatrick phototype scale (Type 3) the increased susceptibility is reflected in a 3 fold greater level of DNA damage in the Patient having the MC1R variant than in the patient without (where 1 CT difference is approximately 3 fold difference in target quantity).

**Table 6: Comparative Analysis of MC1R Variance and mtDNA Deletion for Two Patients**

| | | **MC1R Variant (Amino Acid Position)** | | | | | | | | | | | |
|---|---|---|---|---|---|---|---|---|---|---|---|---|---|
| **PATIENT** | **CT of 3895bp deletion** | **60** | **84** | **92** | **142** | **151** | **160** | **294** | **AGE** | **SEX** | **Hair** | **Skin Tone** | **Effective Use of sunscreens** |
| 303 | 26.1 | 0 | 0 | 1 | 0 | 0 | 0 | 0 | 42 | Female | Black | Olive | Yes |
| 300 | 27.11 | 0 | 0 | 0 | 0 | 0 | 0 | 0 | 45 | Female | Brown | Olive | Yes |

As previously discussed, skin damage caused by UVR is a multifactorial process and, accordingly, it cannot be assumed that an individual with MC1R variants will always have higher damage than an individual without, nor will it be the case that an individual who reports regular sunscreen use will necessarily have lower damage than one who reports no sunscreen use. Combining these two tests provide the health care provider with the insight they require to make more informed recommendations to those in their care.

The following two tables illustrate this point as individuals with variants and without are generally equally represented across the DNA damage spectrum.

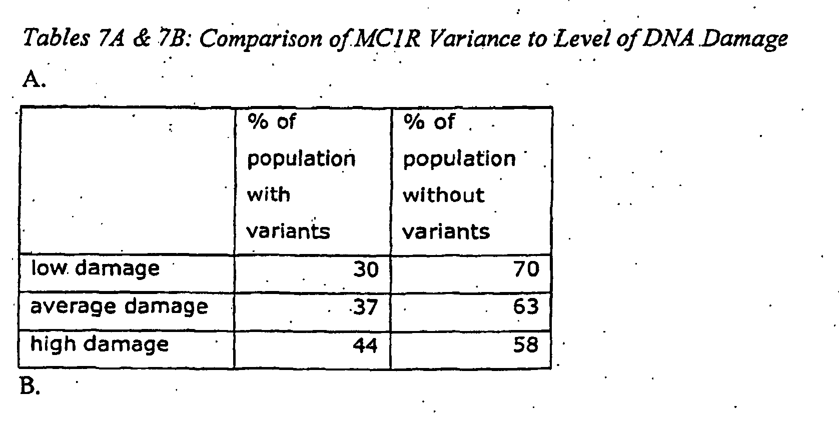

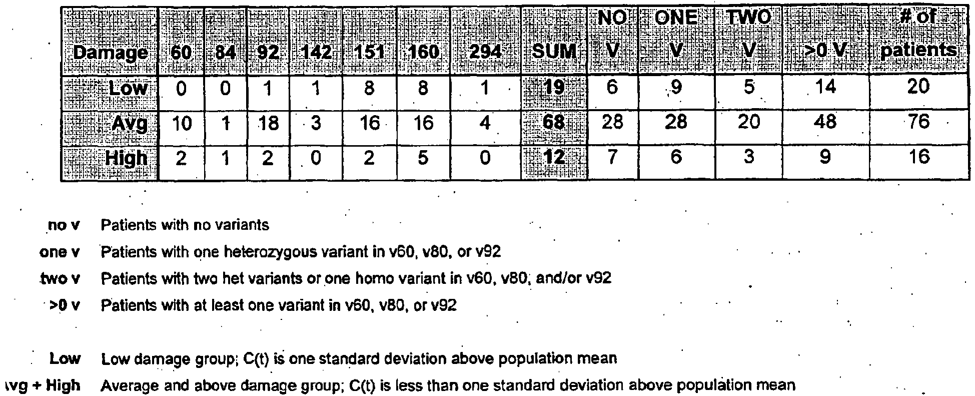

An exception is observed in the next two tables when considering only variants not typically associated with an at risk phenotype. These variants at amino acid positions 60 and 92, are under-represented in the low damage population perhaps indicating that their unique combination of susceptibility to damage as a result of MC1R variants coupled with a lack of visual cues such as fair skin or red hair, indicating increased risk result in a consistently higher level of damage in carriers.

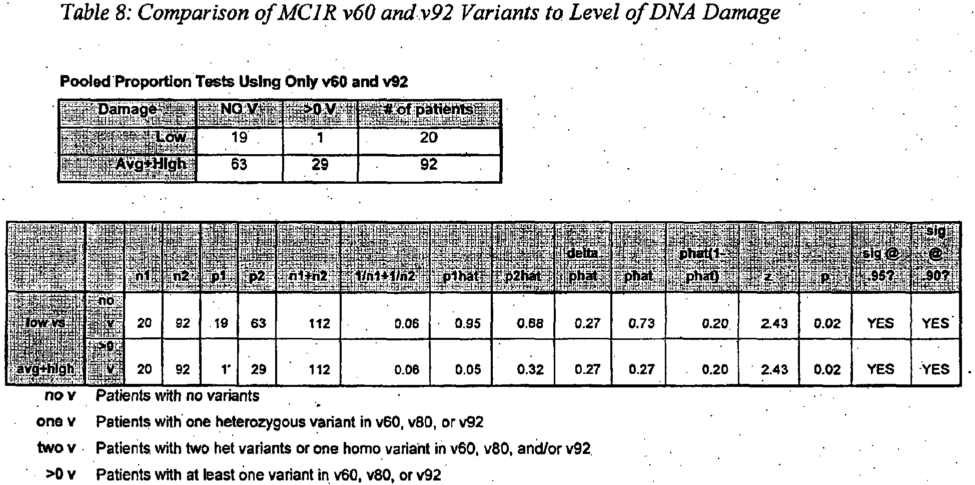

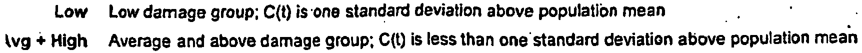

### Example 8: Needle Sampling for Determination of PhotoAge

An additional tool that makes use of a minimally invasive sampling procedure coupled with a mitochondrial marker for the purpose of evaluating UVR exposure is provided for by sampling the skin tissue on the face or head such as the earlobe or near the brow ridge with a small gauge needle (28 gauge), expelling the micro-core into solution, performing nucleic acid extraction and measuring the amount of a mitochondrial target associated with UVR damage such as the 3895bp deletion (see Example 1). This result is then compared against a database of results and classified into an age cohort based upon the level of 3895bp deletion. The database is created by measuring 3895bp deletion levels in multiple individuals in 5 year intervals from ages 0 to 80 years, finding clusters or means for each interval, removing outliers, and correlating the range of level of 3895bp deletion with an age range. For example, CT values from 21.5-23.5 may correlate with an age range of 45-65 years of age. The results of an individual's test would be then compared against their chronological age to determine if they have higher or lower photodamage than other individuals in their age group, and assign them a photoage based on their result.

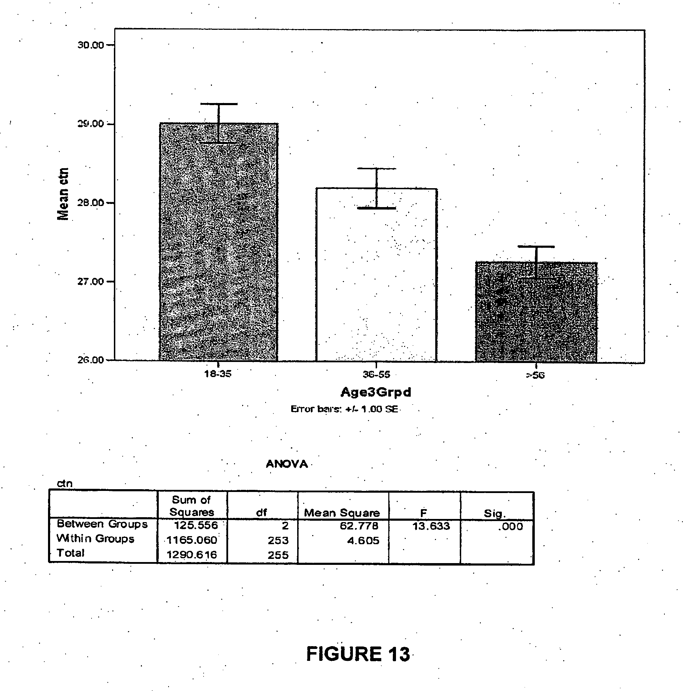

To create this database, microcores were collected from 289 individuals at both their earlobe and brow ridges. As shown in Figure 13, following DNA extraction and quantification of the level of the 3 895bp deletion the database was constructed as described above with age cohorts falling into 3 groups having a significant difference between them (p<0.01).

## Claims

1. A diagnostic method for determining the skin state and genetic predisposition of a subject to UVR damage, comprising:
(a) assaying a skin sample from the subject for a mitochondrial DNA (mtDNA) aberration, wherein the aberration is a 3895 bp mtDNA deletion between nucleotides 546 to 4444 of the mtDNA genome;
(b) assaying a tissue sample from the subject for one or more melanocortin 1 receptor (MC1R) variants, wherein the one or more MC1R variants are selected from the group consisting of D84E, R142H, R151C, R160H, D294H, V60L, and V92M; and
(c) determining the skin state and genetic predisposition of the subject to UVR damage based on the detection of the mtDNA aberration and MC1R variant(s) in the skin and tissue samples, respectively.

2. The method of claim 1, wherein the skin sample was obtained using a non-invasive or minimally invasive skin collecting technique.

3. The method of claim 2, wherein the skin sample was collected using a sterile swab, cotton tip swap, a small gauge needle to collect micro-cores of skin tissue, or a combination thereof.

4. The method of any one of claims 1 to 3, wherein the skin sample was collected from the dermal or epidermal layer of the subject.

5. The method of any one of claims 1 to 4, wherein the skin sample is derived from the heel, nose, inner arm, ear, mouth, scalp, chest, shoulder, buttock, back, face, nape of the neck, hand, head, or a combination thereof.

6. Use of the method of claim 1 for predicting photoaging, UVR damage or skin disease or for determining a prophylactic or therapeutic treatment for preventing or ameliorating photoaging, UVR damage or skin cancer.

7. A method for monitoring a subject's response to a preventative or therapeutic treatment for photoaging, UVR damage or skin disease, comprising:
(a) assaying a first skin sample from the subject for a mitochondrial DNA (mtDNA) aberration, wherein the aberration is a 3895 bp mtDNA deletion between nucleotides 546 to 4444 of the mtDNA genome;
(b) assaying a tissue sample from the subject for one or more melanocortin 1 receptor (MC1R) variants, wherein the one or more MC1R variants are selected from the group consisting of D84E, R142H, R151C, R160H, D294H, V60L, and V92M;
(c) determining the skin state and genetic predisposition of the subject to UVR damage based on the detection of the mtDNA aberration in the first skin sample and the one or more MC1 R variants in the tissue sample;
(d) assaying a second skin sample from the subject for the mtDNA aberration following a prescribed period of time after preventative or therapeutic treatment for photoaging, UVR damage or skin disease;
(e) repeating step (d) at regular intervals over a prescribed period of time; and
(f) comparing the level of the mtDNA aberration between the first skin sample and the skin samples taken at regular intervals to detect changes in the mtDNA aberration, thereby monitoring the effectiveness of the treatment.

8. The method of claim 7, wherein the skin samples were obtained using a non-invasive or minimally invasive skin collecting technique.

9. The method of claim 8, wherein the non-invasive or minimally invasive skin collecting technique comprises obtaining a sufficient amount of the sample to yield ultra low levels of DNA, such as about 0.1 ng of nucleic acid.

10. The method of claim 7, wherein said regular intervals are biweekly or monthly.

11. A method of screening for an effective therapeutic or cosmeceutic agent for the treatment of photoaging, UVR damage or skin disease, comprising;
(a) assaying a first skin sample from a subject for a mitochondrial DNA (mtDNA) aberration, wherein the aberration is a 3895 bp mtDNA deletion between nucleotides 546 to 4444 of the mtDNA genome;
(b) assaying a tissue sample from the subject for one or more melanocortin 1 receptor (MC1R) variants, wherein the one or more MC1R variants are selected from the group consisting of D84E, R142H, R151C, R160H, D294H, V60L, and V92M;
(c) determining the skin state and genetic predisposition of the subject to UVR damage based on the detection of the mtDNA aberration in the first skin sample and the one or more MC1R variants in the tissue sample;
(d) assaying a second skin sample from the subject for the mtDNA aberration, following a prescribed period of time after treatment with the therapeutic or cosmeceutic agent; and
(e) comparing the level of the mtDNA aberration in the first skin sample and the second skin sample against a control to determine the effectiveness of the therapeutic or cosmeceutical agent.

12. The method of claim 11, wherein the first skin sample and second skin sample were obtained using a non-invasive or minimally invasive skin collecting technique.

13. The method of any one of claims 1 to 6 or 7 to 12, wherein the tissue sample for the MC1R assay comprises a skin tissue sample, a buccal tissue sample, a muscle tissue sample, or a nerve tissue sample.

14. A diagnostic kit for determining the skin state and genetic predisposition of a subject to UVR damage, comprising:
(a) material for collecting tissue samples; and
(b) suitable primers, probes and reagents for carrying out melanocortin 1 receptor (MC1 R) genotyping and the detection of a mitochondrial DNA (mtDNA) aberration;
- wherein the mtDNA aberration is a 3895 bp mtDNA deletion between nucleotides 546 to 4444 of the mtDNA genome and wherein the MC1R genotyping comprises the assaying of one or more MC1 R variants selected from the group consisting of D84E, R142H, R151C, R160H, D294H, V60L, and V92M.

## Patentansprüche

1. Diagnostisches Verfahren zur Bestimmung des Zustands der Haut und einer genetischen Prädispositiön eines Subjekts für einen Schaden durch UV-Strahlung, bei dem man:
(a) eine Hautprobe von dem Subjekt auf eine Anomalie der mitochondrialen DNA (mtDNA) untersucht, wobei die Anomalie eine mtDNA-Deletion von 3895 bp zwischen den Nukleotiden 546 bis 4444 des mtDNA-Genoms ist;
(b) eine Gewebeprobe von dem Subjekt auf eine oder mehrere Varianten des Melanocortin-1-Rezeptors (MC1R) untersucht, wobei die eine oder die mehreren MC1R-Variante(n) ausgewählt ist/sind aus der Gruppe bestehend aus D84E, R142H, R151C, R160H, D294H, V60L und V92M; und
(c) den Zustand der Haut und die genetische Prädisposition des Subjekts für einen Schaden durch UV-Strahlung basierend auf dem Nachweis der mtDNA-Anomalie und der MC1R-Variante(n) in der Haut bzw. in den Gewebeproben bestimmt.

2. Verfahren nach Anspruch 1, bei dem die Hautprobe unter Verwendung eines nicht-invasiven oder eines minimal-invasiven Hautsammelverfahrens erhalten wurde.

3. Verfahren nach Anspruch 2, bei dem die Hautprobe unter Verwendung eines sterilen Tupfers, eines Tupfers mit Wattespitze, einer Nadel mit geringem Gauge-Wert zur Sammlung von Mikro-Hautgewebeproben, oder einer Kombination derselben gesammelt wurde.

4. Verfahren nach einem der Ansprüche 1 bis 3, bei dem die Hautprobe aus der dermalen oder der epidermalen Schicht des Subjekts gewonnen wurde.

5. Verfahren nach einem der Ansprüche 1 bis 4, bei dem die Hautprobe von der Ferse, der Nase, der Innenseite des Arms, dem Ohr, dem Mund, der Kopfhaut, der Brust, der Schulter, dem Gesäß, dem Rücken, dem Gesicht, dem Nacken, der Hand, dem Kopf oder einer Kombination davon erhalten wird.

6. Verwendung des Verfahrens nach Anspruch 1 zur Vorhersage einer lichtbedingten Hautalterung (Photoaging), eines Schadens durch UV-Strahlung oder einer Hauterkrankung oder zur Bestimmung einer prophylaktischen oder therapeutischen Behandlung zur Vermeidung oder Abschwächung einer lichtbedingten Hautalterung (Photoaging), eines Schadens durch UV-Strahlung oder von Hautkrebs.

7. Verfahren zur Überprüfung der Antwort eines Subjekts auf eine präventive oder therapeutische Behandlung gegen eine lichtbedingte Hautalterung (Photoaging), einen Schaden durch UV-Strahlung oder eine Hauterkrankung, bei dem man
(a) eine erste Hautprobe von dem Subjekt auf eine Anomalie der mitochondrialen DNA (mtDNA) untersucht, wobei die Anomalie eine mtDNA-Deletion von 3895 bp zwischen den Nukleotiden 546 bis 4444 des mtDNA-Genoms ist;
(b) eine Gewebeprobe von dem Subjekt auf eine oder mehrere Varianten des Melanocortin-1-Rezeptors (MC1R) untersucht, wobei die eine oder die mehreren MC1R-Variante(n) ausgewählt ist/sind aus der Gruppe bestehend aus D84E, R142H, R151C, R160H, D294H, V60L und V92M; und
(c) den Zustand der Haut und die genetische Prädisposition des Subjekts für einen Schaden durch UV-Strahlung basierend auf dem Nachweis der mtDNA-Anomalie in der ersten Gewebeprobe und der einen oder der mehreren MC1R-Variante(n) in der Gewebeprobe bestimmt;
(d) nach einem vorbestimmten Zeitraum nach der präventiven oder therapeutischen Behandlung gegen eine lichtbedingte Hautalterung (Photoaging), einen Schaden durch UV-Strahlung oder eine Hauterkrankung eine zweite Hautprobe von dem Subjekt auf die mtDNA-Anomalie untersucht;
(e) Schritt (d) in gleichmäßigen Intervallen über einen vorbestimmten Zeitraum wiederholt; und
(f) das Ausmaß der mtDNA-Anomalie zwischen der ersten Hautprobe und den in regelmäßigen Intervallen entnommenen Hautproben vergleicht, um Änderungen in der mtDNA-Anomalie nachzuweisen, wodurch die Wirksamkeit der Behandlung überprüft wird.

8. Verfahren nach Anspruch 7, bei dem die Hautproben unter Verwendung eines nicht-invasiven oder eines minimal-invasiven Hautsammelverfahrens erhalten wurden.

9. Verfahren nach Anspruch 8, bei dem das nicht-invasive oder das minimal-invasive Hautsammelverfahren Schritte umfasst, bei denen eine ausreichende Menge der Probe erhalten wird, um ultrageringe DNA-Mengen bereitzustellen, wie z.B. 0,1 ng Nukleinsäure.

10. Verfahren nach Anspruch 7, bei dem die regelmäßigen Intervalle zweimal wöchentlich oder monatlich sind.

11. Verfahren zum Screening nach einem wirksamen therapeutischen oder kosmetisch-pharmazeutischen Mittel zur Behandlung einer lichtbedingten Hautalterung (Photoaging), eines Schadens durch UV-Strahlung oder einer Hauterkrankung, bei dem man
(a) eine erste Hautprobe von einem Subjekt auf eine Anomalie der mitochondrialen DNA (mtDNA) untersucht, wobei die Anomalie eine mtDNA-Deletion von 3895 bp zwischen den Nukleotiden 546 bis 4444 des mtDNA-Genoms ist;
(b) eine Gewebeprobe von dem Subjekt auf eine oder mehrere Varianten des Melanocortin-1-Rezeptors (MC1R) untersucht, wobei die eine oder die mehreren MC1R-Variante(n) ausgewählt ist/sind aus der Gruppe bestehend aus D84E, R142H, R151C, R160H, D294H, V60L und V92M; und
(c) den Zustand der Haut und die genetische Prädisposition des Subjekts für einen Schaden durch UV-Strahlung basierend auf dem Nachweis der mtDNA-Anomalie in der ersten Gewebeprobe und der einen oder mehreren MC1R-Variante(n) in der Gewebeprobe bestimmt;
(d) nach einem vorbestimmten Zeitraum nach der therapeutischen oder kosmetisch-pharmazeutischen Behandlung eine zweite Hautprobe von dem Subjekt auf die mtDNA-Anomalie untersucht; und
(e) das Ausmaß der mtDNA-Anomalie in der erste Hautprobe und der zweiten Hautprobe mit einer Kontrolle vergleicht, um die Wirksamkeit des therapeutischen oder des kosmetisch-pharmazeutischen Mittels zu bestimmen.

12. Verfahren nach Anspruch 11, bei dem die erste Hauptprobe und die zweite Hautprobe unter Verwendung eines nicht-invasiven oder eines minimal-invasiven Hautsammelverfahrens erhalten wurden.

13. Verfahren nach einem der Ansprüche 1 bis 6 oder 7 bis 12, bei dem die Gewebeprobe für den MC1R-Assay eine Hautgewebeprobe, eine Wangengewebeprobe, eine Muskelgewebeprobe oder eine Nervengewebeprobe umfasst.

14. Diagnostischer Kit zur Bestimmung des Zustands der Haut und einer genetischen Prädisposition eines Subjekts für einen Schaden durch UV-Strahlung, umfassend:
(a) Material zum Sammeln von Gewebeproben; und
(b) geeignete Primer, Sonden und Reagenzien zur Durchführung einer Genotypisierung des Melanocortin-1-Rezeptors (MC1R) und des Nachweises einer Anomalie der mitochondrialen DNA (mtDNA);
wobei die mtDNA-Anomalie eine mtDNA-Deletion von 3895 bp zwischen den Nukleotiden 546 und 4444 des mtDNA-Genoms ist, und wobei die MC1R-Genotypisierung die Untersuchung von einer oder von mehreren MC1R-Variante(n) ausgewählt aus der Gruppe bestehend aus D84E, R142H, R151C, R160H, D294H, V60L und V92M umfasst.

## Revendications

1. Procédé diagnostique destiné à déterminer l'état de la peau et une prédisposition génétique d'un patient à des lésions dues à un rayonnement ultraviolet, comprenant les étapes consistant à :
(a) analyser un échantillon de peau en provenance du patient pour déterminer une aberration de l'ADN mitochondrial (ADNmt), dans lequel l'aberration est une délétion de 3895 pb de l'ADNmt entre les nucléotides 546 à 4444 du génome d'ADNmt ;
(b) analyser un échantillon de tissu en provenance du patient pour déterminer un ou plusieurs variant(s) du récepteur de la mélanocortine de type 1 (MC1R), dans lequel le ou les variant(s) du MC1R est/sont sélectionné(s) dans le groupe constitué par D84E, R142H, R151C, R160H, D294H, V60L et V92M ; et
(c) déterminer l'état de la peau et une prédisposition génétique d'un patient à des lésions dues à un rayonnement ultraviolet sur la base de la détection de l'aberration de l'ADNmt et du ou des variant(s) du MC1R dans les échantillons respectifs de peau et de tissu.

2. Procédé selon la revendication 1, dans lequel l'échantillon de peau a été obtenu en utilisant une technique de prélèvement de peau non invasive ou très peu invasive.

3. Procédé selon la revendication 2, dans lequel l'échantillon de peau a été prélevé en utilisant un tampon stérile, un coton-tige, une aiguille de petit calibre pour prélever des micro-noyaux de tissu de peau, ou une combinaison de ceux-ci.

4. Procédé selon l'une quelconque des revendications 1 à 3, dans lequel l'échantillon de peau a été prélevé à partir de la couche dermique ou épidermique du patient.

5. Procédé selon l'une quelconque des revendications 1 à 4, dans lequel l'échantillon de peau est obtenu à partir du talon, du nez, de l'intérieur du bras, de l'oreille, de la bouche, du cuir chevelu, de la poitrine, de l'épaule, des fesses, du dos, du visage, de la nuque du cou, de la main, de la tête, ou d'une combinaison de ceux-ci.

6. Utilisation du procédé selon la revendication 1, destiné à prévoir un photo-vieillissement, des lésions dues à un rayonnement ultraviolet ou une maladie de la peau, ou à déterminer un traitement prophylactique ou thérapeutique destiné à prévenir ou à améliorer un photo-vieillissement, des lésions dues à un rayonnement ultraviolet ou un cancer de la peau.

7. Procédé destiné à surveiller la réponse d'un patient à un traitement préventif ou thérapeutique d'un photo-vieillissement, de lésions dues à un rayonnement ultraviolet ou d'une maladie de la peau, comprenant les étapes consistant à :
(a) analyser un premier échantillon de peau en provenance du patient pour déterminer une aberration de l'ADN mitochondrial (ADNmt), dans lequel l'aberration est une délétion de 3895 pb de l'ADNmt entre les nucléotides 546 à 4444 du génome d'ADNmt ;
(b) analyser un prélèvement de tissu en provenance du patient pour déterminer un ou plusieurs variant(s) du récepteur de la mélanocortine de type 1 (MC1R), dans lequel le ou les variant(s) du MC1R est/sont sélectionné(s) dans le groupe constitué par D84E, R142H, R151C, R160H, D294H, V60L et V92M ;
(c) déterminer l'état de la peau et une prédisposition génétique d'un patient à des lésions dues à un rayonnement ultraviolet sur la base de la détection de l'aberration de l'ADNmt dans le premier échantillon de peau et du ou des variant(s) du MC1R dans l'échantillon de tissu ;
(d) analyser un second échantillon de peau en provenance du patient pour déterminer une aberration de l'ADNmt suite à une période de temps prescrite après un traitement préventif ou thérapeutique d'un photo-vieillissement, de lésions dues à un rayonnement ultraviolet ou d'une maladie de la peau ;
(e) répéter l'étape (d) à intervalles réguliers sur une période de temps prescrite ; et
(f) comparer le niveau de l'aberration de l'ADNmt entre le premier échantillon de peau et les échantillons de peau prélevés à intervalles réguliers de façon à détecter des modifications de l'aberration de l'ADNmt, en surveillant de ce fait l'efficacité du traitement.

8. Procédé selon la revendication 7, dans lequel les échantillons de peau ont été obtenus en utilisant une technique de prélèvement de peau non invasive ou très peu invasive.

9. Procédé selon la revendication 8, dans lequel la technique de prélèvement de peau non invasive ou très peu invasive comprend l'obtention d'une quantité d'échantillon suffisante pour obtenir des niveaux ultrafaibles d'ADN, par exemple 0,1 ng environ d'acide nucléique.

10. Procédé selon la revendication 7, dans lequel lesdits intervalles réguliers sont bihebdomadaires ou mensuels.

11. Procédé destiné à passer au crible un agent thérapeutique ou cosméceutique efficace destiné au traitement d'un photo-vieillissement, de lésions dues à un rayonnement ultraviolet ou d'une maladie de la peau, comprenant les étapes consistant à ;
(a) analyser un premier échantillon de peau en provenance d'un patient pour déterminer une aberration de l'ADN mitochondrial (ADNmt), dans lequel l'aberration est une délétion de 3895 pb de l'ADNmt entre les nucléotides 546 à 4444 du génome d'ADNmt ;
(b) analyser un prélèvement de tissu en provenance du patient pour déterminer un ou plusieurs variant(s) du récepteur de la mélanocortine de type 1 (MC1R), dans lequel le ou les variant(s) du MC1R est/sont sélectionné(s) dans le groupe constitué par D84E, R142H, R151C, R160H, D294H, V60L et V92M ; et
(c) déterminer l'état de la peau et une prédisposition génétique d'un patient à des lésions dues à un rayonnement ultraviolet sur la base de la détection de l'aberration de l'ADNmt dans le premier échantillon de peau et du ou des variants du MC1R dans l'échantillon de tissu ;
(d) analyser un second échantillon de peau en provenance du patient pour déterminer une aberration de l'ADNmt suite à une période de temps prescrite après un traitement avec l'agent thérapeutique ou cosméceutique ; et
(e) comparer le niveau de l'aberration de l'ADNmt dans le premier échantillon de peau et le second échantillon de peau à l'aide d'un contrôle de façon à déterminer l'efficacité de l'agent thérapeutique ou cosméceutique.

12. Procédé selon la revendication 11, dans lequel le premier échantillon de peau et le second échantillon de peau ont été obtenus en utilisant une technique de prélèvement de peau non invasive ou très peu invasive.

13. Procédé selon l'une quelconque des revendications 1 à 6 ou 7 à 12, dans lequel l'échantillon de tissu destiné à l'analyse de MC1R comprend un échantillon de tissu de peau, un échantillon de tissu buccal, un échantillon de tissu musculaire, ou un échantillon de tissu nerveux.

14. Kit diagnostique destiné à déterminer l'état de la peau et une prédisposition génétique d'un patient à des lésions dues à un rayonnement ultraviolet, comprenant :
(a) un matériau destiné à prélever des échantillons de tissu ; et
(b) des amorces, des sondes et des réactifs appropriés pour procéder à un génotypage du récepteur de la mélanocortine de type 1 (MC1R) et à la détection d'une aberration de l'ADN mitochondrial (ADNmt) ;
- dans lequel l'aberration de l'ADNmt est une délétion de 3895 pb de l'ADNmt entre les nucléotides 546 à 4444 du génome d'ADNmt, et dans lequel le génotypage du MC 1 R comprend l'analyse d'un ou de plusieurs variants du MC 1 R sélectionnés dans le groupe constitué par D84E, R142H, R151C, R160H, D294H, V60L, et V92M.
